# EUROPEAN PATENT APPLICATION

(11) **EP 4 819 058 A1**
(43) Date of publication of application: **07.10.2026**
(21) Application number: 26169702.3
(22) Date of filing: 31.03.2026
(51) Int. Cl.: G06V 10/12, A61B 6/03, G06V 10/94

(54) **X-RAY CT APPARATUS, CONTROL METHOD BY X-RAY CT APPARATUS AND STORAGE MEDIUM**

(30) Priority: 01.04.2025 JP 2025060677; 09.02.2026 JP 2026019162
(71) Applicant: Canon Medical Systems Corporation, Tochigi 324-0036 (JP)
(72) Inventor: TAKEI, Yuma, Otawara-shi, 324-0036 (JP); TEZUKA, Akira, Otawara-shi, 324-0036 (JP)
(74) Representative: Marks & Clerk LLP

(57) **Abstract**

An X-ray CT apparatus (1) according to an embodiment includes a scanner unit (20), a support unit (103), a detection unit (56), a detection control unit (55), and an operation control unit (51). The scanner unit has an imaging system. The support unit supports the scanner unit and moves the scanner unit in the vertical direction. The detection unit defines at least part of an area between an outer periphery of an opening into which a subject is inserted and the subject as a detection area and detect an object present in the detection area. The detection control unit performs control to define an area in which the movement of the subject is restricted as a non-detection area. The operation control unit stops the operation of the scanner unit when it is detected that the object is present in the detection area excluding the non-detection area.

## Description

### FIELD

Embodiments described herein relate generally to an X-ray CT apparatus, a control method by the X-ray CT apparatus, and a storage medium.

### BACKGROUND

There have been developed X-ray computed tomography (CT) apparatuses in which a scanner into which a subject is inserted is tilted or vertically moved by being supported by a stand. Such X-ray CT apparatuses can image a subject in a standing position (standing position imaging) or a subject sitting in a chair or a wheelchair (sitting position imaging).

### SUMMARY OF THE INVENTION

According to a first aspect, an X-ray CT apparatus is provided. The apparatus includes a scanner unit having an imaging system; a support unit that supports the scanner unit and configured to move the scanner unit in a vertical direction; a detection unit configured to define at least part of an area between an outer periphery of an opening into which a subject is inserted and the subject as a detection area and detect that an object is present in the detection area; a detection control unit configured to perform control to define the detection area included in an area in which movement of the subject is restricted as a non-detection area; and an operation control unit configured to stop an operation of the scanner unit when it is detected that the object is present in the detection area excluding the non-detection area while the scanner unit is operating.

The detection control unit may perform control to define an area corresponding to an installation position of a subject support member for supporting the subject as the non-detection area in standing position imaging or sitting position imaging of the subject.

The X-ray CT apparatus may further include a plurality of optical sensors provided to the scanner unit and configured to detect the object. The detection unit may detect that the object is present in the detection area based on a result of detection by each of the optical sensors, and may perform control to disable at least one optical sensor present in the non-detection area according to the installation position of the subject support member for supporting the subject out of the optical sensors.

The detection control unit may perform control to define an area including the optical sensor closest to the installation position of the subject support member alone out of the optical sensors as the non-detection area.

The detection control unit may perform control to define the area behind the installation position of the subject support member out of the area including the optical sensors as the non-detection area.

The optical sensors may be divided into at least two group areas according to positions at which the optical sensors are provided. The detection control unit may perform control to define the group area closest to the installation position of the subject support member out of the area including the optical sensors as the non-detection area.

The X-ray CT apparatus may further include a display control unit configured to display at least one of the detection area and the non-detection area on a display medium.

The display medium may be a display unit of a display apparatus provided to the support unit. The display control unit may display at least one of the detection area and the non-detection area on the display unit.

The display medium may be at least one of an exterior of the scanner unit and a floor surface of an examination room in which the X-ray CT apparatus is installed. The display control unit may display at least one of the detection area and the non-detection area on the exterior or the floor surface using at least one of a plurality of light-emitting diodes (LEDs) provided to the exterior, a projector configured to project an image onto the exterior, and a display apparatus embedded in the floor surface.

The detection control unit may define an area opposite to a direction in which the subject is capable of moving during imaging as the non-detection area.

According to a second aspect, an X-ray CT apparatus is provided. The apparatus includes a scanner unit having an imaging system; a support unit that supports the scanner unit and configured to move the scanner unit in a vertical direction; a detection unit configured to define at least part of an area between an outer periphery of an opening into which a subject is inserted and the subject as a detection area and detect that the subject is present in the detection area or that an object other than the subject is present in the detection area; and an operation control unit configured to stop an operation of the scanner unit when it is detected that the subject is present in the detection area while the scanner unit is operating. The operation control unit does not perform control to stop the operation of the scanner unit when it is detected that the object other than the subject is present in the detection area.

The X-ray CT apparatus may further include a plurality of optical sensors configured to detect the object. The optical sensors may each include a light emitter configured to emit a laser beam toward a light receiver, and the light receiver configured to receive the emitted laser beam. The detection unit may determine that at least one of the subject and the object other than the subject is detected in the detection area when at least one of the light receivers fails to receive the laser beam. The detection unit may identify whether the subject or the object other than the subject is detected in the detection area based on the number of light receivers that determine that the laser beam adjacent to the light receivers fails to be received.

The light emitters of the optical sensors may each emit a plurality of the laser beams such that the laser beams intersect between each of the light emitters and each of the light receivers in the vertical direction. The detection unit may identify whether the subject or the object other than the subject is detected in the detection area based on a combination of the light receivers that fail to receive the laser beams.

The detection unit may detect the object other than the subject based on the combination of the light receivers that fail to receive the laser beams in a non-detection area other than the detection area. The X-ray CT apparatus may further include a presentation unit configured to present an area around the scanner unit to an operator of the X-ray CT apparatus by dividing the area into "an area serving as the detection area and in which the object other than the subject is detected", "an area serving as the detection area and in which the object other than the subject is not detected", "an area serving as the non-detection area and in which the object other than the subject is detected", and "an area serving as the non-detection area and in which the object other than the subject is not detected".

According to a third aspect, there is provided a control method by an X-ray CT apparatus which includes a scanner unit having an imaging system, and a support unit that supports the scanner unit and configured to move the scanner unit in a vertical direction. The control method includes defining at least part of an area between an outer periphery of an opening into which a subject is inserted and the subject as a detection area and detecting an object present in the detection area; performing control to define an area in which movement of the subject is restricted as a non-detection area; and stopping an operation of the scanner unit when it is detected that the object is present in the detection area excluding the non-detection area while the scanner unit is operating.

According to a fourth aspect, a non-transitory computer readable medium including instructions is provided. The instructions cause a computer of an X-ray CT apparatus including a scanner unit having an imaging system, and a support unit that supports the scanner unit and configured to move the scanner unit in a vertical direction, to execute defining at least part of an area between an outer periphery of an opening into which a subject is inserted and the subject as a detection area and detecting an object present in the detection area; performing to define an area in which movement of the subject is restricted as a non-detection area; and stopping an operation of the scanner unit when it is detected that the object is present in the detection area excluding the non-detection area while the scanner unit is operating.

### BRIEF DESCRIPTION OF THE DRAWINGS

FIG. 1 is a block diagram of an example of the configuration of an X-ray CT apparatus according to a first embodiment;
FIG. 2 is a schematic of an example of the appearance of the X-ray CT apparatus according to the first embodiment;
FIG. 3 is a perspective view of an example of the X-ray CT apparatus that images a subject in a supine position according to the first embodiment;
FIG. 4 is a perspective view of an example of the X-ray CT apparatus that images the subject in a standing position according to the first embodiment;
FIG. 5 is a top view of an example of a scanner according to the first embodiment;
FIG. 6 is a top view of an example of a detection area when contrast-enhanced imaging is not performed according to the first embodiment;
FIG. 7 is a view of an example of installation positions of a pole according to the first embodiment;
FIG. 8 is a top view of an example of the detection area when the pole is installed at a first installation position according to the first embodiment;
FIG. 9 is a top view of an example of the detection area when the pole is installed at a second installation position according to the first embodiment;
FIG. 10 is a top view of an example of the detection area when the pole is installed at a third installation position according to the first embodiment;
FIG. 11 is a view of an example of a display screen for notifying an operator of the detection area according to the first embodiment;
FIG. 12A is a flowchart of an example of processing performed by the X-ray CT apparatus according to the first embodiment;
FIG. 12B is a flowchart of an example of the processing performed by the X-ray CT apparatus according to the first embodiment;
FIG. 13 is a block diagram of an example of the configuration of the X-ray CT apparatus according to a second embodiment;
FIG. 14 is a diagram for explaining an example of processing for determining whether the subject or an object other than the subject is detected according to the second embodiment;
FIG. 15 is a diagram for explaining an example of the processing for determining whether the subject or an object other than the subject is detected according to the second embodiment;
FIG. 16 is a flowchart of an example of the processing performed by the X-ray CT apparatus according to the second embodiment;
FIG. 17 is a top view of an example of the detection area determined according to front setting according to a second modification;
FIG. 18 is a top view for explaining an example of the positional relation between the installation position of the pole and an area to be enabled according to a fourth modification;
FIG. 19 is a top view for explaining an example of the positional relation between the installation position of the pole and laser sensors to be enabled according to a fifth modification;
FIG. 20 is a top view for explaining an example of the positional relation between the installation position of the pole and the laser sensors to be enabled according to a sixth modification;
FIG. 21 is a view of an example of a display screen for notifying the operator of a tube-placeable region according to a seventh modification;
FIG. 22 is a diagram for explaining an example of the processing for determining whether the subject or an object other than the subject is detected according to an eighth modification; and
FIG. 23 is a flowchart of an example of the processing performed by the X-ray CT apparatus according to the eighth modification.

### DETAILED DESCRIPTION

An X-ray CT apparatus according to an embodiment includes a scanner unit, a support unit, and processing circuitry. The scanner unit has an imaging system. The support unit supports the scanner unit and moves the scanner unit in the vertical direction. The processing circuitry performs control to define at least part of an area between an outer periphery of an opening into which a subject is inserted and the subject as a detection area and detect an object present in the detection area and to define an area in which the movement of the subject is restricted as a non-detection area, and stops the operation of the scanner unit when it is detected that the object is present in the detection area excluding the non-detection area while the scanner unit is operating.

### First Embodiment

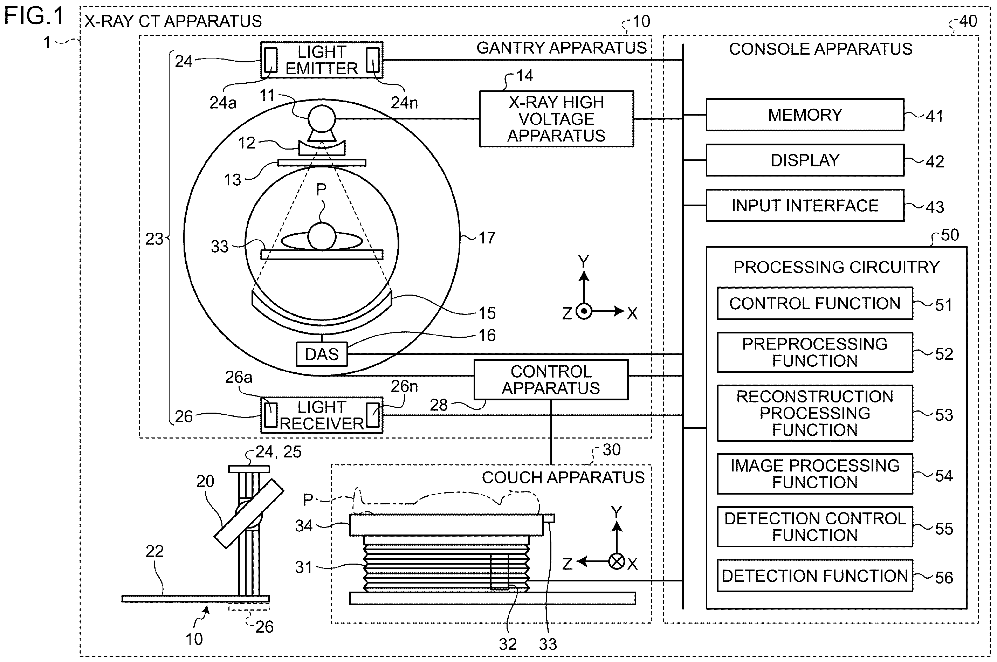

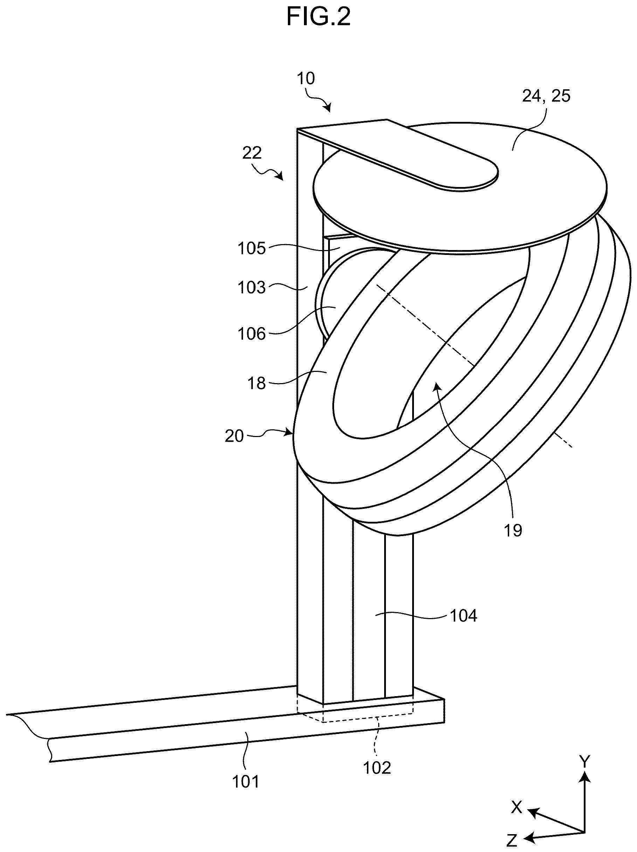

FIG. 1 is a block diagram of an example of the configuration of an X-ray CT apparatus 1 according to a first embodiment. FIG. 2 is a schematic of an example of the appearance of the X-ray CT apparatus 1 according to the first embodiment.

The X-ray CT apparatus 1 includes, for example, a gantry apparatus 10, a couch apparatus 30, and a console apparatus 40. While FIG. 1 illustrates both a view of the gantry apparatus 10 from a Z-axis direction and a view from an X-axis direction for the sake of explanation, one gantry apparatus 10 is provided in an actual configuration.

In the present embodiment, the Z-axis direction (front-back direction) is defined as the direction of the rotation axis of a rotating frame 17 in a non-tilted state and along the horizontal direction or the longitudinal direction of a couchtop 33 of the couch apparatus 30. The X-axis direction (circumferential direction) is defined as the direction orthogonal to the Z-axis direction and horizontal to the floor surface. A Y-axis direction (vertical direction) is defined as the direction orthogonal to the Z-axis direction and perpendicular to the floor surface.

The gantry apparatus 10 in the X-ray CT apparatus 1 includes, for example, a scanner 20, a scanner drive apparatus 22, light emitters 24a to 24n, and a control apparatus 28. The light emitters 24a to 24n are supported by a support member 25. Light receivers 26a to 26n are provided on the floor surface where the gantry apparatus 10 is installed.

The scanner 20 is supported by the scanner drive apparatus 22. The scanner drive apparatus 22 moves the scanner 20 in the vertical and horizontal directions and tilts the scanner 20 to change the orientation of the scanner 20. The control apparatus 28 controls the operations of the scanner drive apparatus 22.

The scanner 20 is an example of a scanner unit. The scanner 20 includes an X-ray tube 11, a wedge 12, a collimator 13, an X-ray high voltage apparatus 14, an X-ray detector 15, a data acquisition system (hereinafter referred to as DAS) 16, the rotating frame 17, and a cover 18. The X-ray tube 11, the wedge 12, the collimator 13, the X-ray high voltage apparatus 14, the X-ray detector 15, the DAS 16, and the rotating frame 17 are housed in the cover 18.

The X-ray tube 11 generates X-rays by outputting thermionic electrons from a cathode (filament) to an anode (target) due to the application of high voltage from the X-ray high voltage apparatus 14. The X-ray tube 11 outputs X-rays to a subject P. The X-ray tube 11 includes a vacuum tube. For example, the X-ray tube 11 is a rotating anode X-ray tube that generates X-rays by outputting thermionic electrons to a rotating anode.

The wedge 12 is a filter for adjusting the dose of X-rays (radiation dose) output from the X-ray tube 11 to the subject (imaging target) P. The wedge 12 attenuates the X-rays passing therethrough such that the distribution of the dose of X-rays output from the X-ray tube 11 to the subject P is a predetermined distribution. The wedge 12 is also called a wedge filter or bow-tie filter. The wedge 12 is made of aluminum machined to have a predetermined target angle and a predetermined thickness, for example.

The collimator 13 is a mechanism to collimate the irradiation range of X-rays transmitted through the wedge 12. The collimator 13 collimates the X-ray irradiation range by forming a slit with a combination of a plurality of lead plates, for example. The collimator 13 may also be referred to as an X-ray diaphragm. The collimator 13 may be an active collimator the collimation range of which is mechanically drivable.

The X-ray high voltage apparatus 14 includes, for example, a high voltage generation apparatus and an X-ray control apparatus. The high voltage generation apparatus has electric circuitry including a transformer, a rectifier, and other components and generates high voltage to be applied to the X-ray tube 11. The X-ray control apparatus controls the output voltage of the high voltage generation apparatus according to the dose of X-rays to be generated by the X-ray tube 11.

The high voltage generation apparatus may raise the voltage by the transformer described above or an inverter. The X-ray high voltage apparatus 14 may be provided to the rotating frame 17 or a fixed frame (not illustrated) of the gantry apparatus 10.

The X-ray detector 15 detects the intensity of X-rays generated by the X-ray tube 11, transmitted through the subject P, and incident on the X-ray detector 15. The X-ray detector 15 outputs electrical signals (or optical signals or the like) corresponding to the detected intensity of X-rays to the DAS 16. The X-ray detector 15 includes a plurality of X-ray detector element rows, for example. The X-ray detector element rows are each composed of a plurality of X-ray detector elements arrayed in a channel direction along an arc centered at the focus of the X-ray tube 11. The X-ray detector element rows are arrayed in a slice direction (column or row direction).

The X-ray detector 15 is an indirect detector including, for example, a grid, a scintillator array, and an optical sensor array.

The scintillator array includes a plurality of scintillators. Each scintillator includes a scintillator crystal. The scintillator crystal outputs an amount of light corresponding to the intensity of incident X-rays.

The grid is disposed on the surface of the scintillator array on which the X-rays are incident and includes an X-ray shielding plate having a function to absorb scattered X-rays. The grid may also be referred to as a collimator (one-dimensional collimator or two-dimensional collimator).

The optical sensor array includes an optical sensor, such as a photomultiplier tube (PMT). The optical sensor array outputs electrical signals corresponding to the amount of light output from the scintillator. The X-ray detector 15 may be a direct conversion detector including semiconductor elements that convert incident X-rays into electrical signals.

The DAS 16 includes, for example, an amplifier and an A/D converter. The amplifier performs amplification processing on electrical signals output from each of the X-ray detector elements of the X-ray detector 15. The A/D converter converts electrical signals into digital signals. The DAS 16 outputs detection data based on the digital signals to the console apparatus 40.

The rotating frame 17 is an annular member that oppositely supports the X-ray tube 11, the wedge 12, and the collimator 13, and the X-ray detector 15. The rotating frame 17 is rotatably supported by the fixed frame about the subject P introduced therein. The rotating frame 17 further supports the DAS 16.

The detection data output by the DAS 16 is transmitted by optical communications from a transmitter provided to the rotating frame 17 and including light-emitting diodes (LEDs) to a receiver provided to a non-rotating part (e.g., fixed frame) of the gantry apparatus 10 and including photodiodes. The detection data is transferred to the console apparatus 40 by the receiver.

The method for transmitting the detection data from the rotating frame 17 to the non-rotating part is not limited to the method using optical communications described above and may be any non-contact transmission method. The rotating frame 17 is not limited to an annular member and may be an arm-like member as long as it can support and rotate the X-ray tube 11 and other components.

The cover 18 has a central opening 19. The central opening 19 is an opening into which the subject P is inserted. The rotating frame 17 is disposed inside the cover 18 in a manner surrounding the central opening 19. The rotating frame 17 rotates around the central opening 19.

The X-ray CT apparatus 1 is a rotate/rotate-type X-ray CT apparatus (third-generation CT) in which both the X-ray tube 11 and the X-ray detector 15 are supported by the rotating frame 17 and rotate around the subject P, for example. The X-ray CT apparatus 1 is not limited thereto and may be a stationary/rotate-type X-ray CT apparatus (fourth-generation CT) in which a plurality of annularly arrayed X-ray detector elements are fixed to a fixed frame and the X-ray tube 11 rotates around the subject P.

The scanner drive apparatus 22 includes, for example, a support column 103, a rail 104, a slider 105, and a tilt mechanism 106.

The scanner drive apparatus 22 may further include a base 101 and a horizontal movement apparatus 102. FIG. 2 illustrates an aspect further including the base 101 and the horizontal movement apparatus 102. In the following description, the scanner drive apparatus 22 includes the base 101 and the horizontal movement apparatus 102 in principle.

In the example in FIG. 2, the base 101 has a linear support structure extending in the horizontal direction. The base 101 is fixed to the floor surface of a treatment room by bolts, for example. The horizontal movement apparatus 102 is provided to the base 101, and the support column 103 is mounted upright on the horizontal movement apparatus 102.

The support column 103 is a member extending in the vertical direction, for example. The support column 103 is an example of a support unit. If the scanner drive apparatus 22 does not include the base 101, the support column 103 may be fixed to the floor surface of the treatment room by bolts, for example. The horizontal movement apparatus 102 moves the support column 103 serving as the mounted component in the horizontal direction under the control of the control apparatus 28.

The support column 103 is provided with the rail 104. The rail 104 is disposed along the extending direction (vertical direction) of the support column 103. The rail 104 is provided with the slider 105. The slider 105 is movable along the rail 104 under the control of the control apparatus 28.

The slider 105 is provided with the tilt mechanism 106, and the tilt mechanism 106 is provided with the scanner 20. The tilt mechanism 106 can tilt the scanner 20 about the rotation axis under the control of the control apparatus 28. The tilt mechanism 106 tilts the scanner 20, thereby changing the orientation of the scanner 20. The scanner drive apparatus 22 moves the support column 103 by the horizontal movement apparatus 102, thereby moving the scanner 20 in the horizontal direction.

The scanner drive apparatus 22 causes the slider 105 to move along the rail 104, thereby moving the scanner 20 in the vertical direction. The scanner drive apparatus 22 tilts the scanner 20 about the rotation axis by the tilt mechanism 106. The scanner drive apparatus 22 moves the scanner 20, thereby relatively moving the scanner 20 and the subject P.

The light emitters 24a to 24n (hereinafter simply referred to as the light emitters 24 when they are not particularly distinguished) are provided to the scanner 20. For example, the light emitters 24a to 24n are provided along the circular area obtained by projecting the central opening 19 of the scanner 20 (hereinafter simply referred to as the central opening 19) onto the support member 25.

The light emitters 24a to 24n emit laser beams downward. While the light emitters 24a to 24n according to the present embodiment output visible beams to the light receivers 26a to 26n (hereinafter simply referred to as the light receivers 26 when they are not particularly distinguished), the light emitters 24 may output infrared light to the light receivers 26.

The light receivers 26a to 26n are provided on the floor surface along the circular area obtained by projecting the central opening 19 onto the floor surface. The light receivers 26a to 26n receive laser beams 32a to 32n emitted from the light emitters 24a to 24n, respectively, and transmit the reception results to the control apparatus 28.

The light emitters 24a to 24n and the light receivers 26a to 26n constitute laser sensors 23a to 23n. A laser sensor 23 is an example of an optical sensor. Specifically, the light emitters 24a to 24n are paired with the light receivers 26a to 26n provided on a floor surface 5, respectively, and a pair of the light emitter 24 and the light receiver 26 constitute one laser sensor 23.

In the example illustrated in FIG. 1, the light emitter 24a and the light receiver 26a facing the light emitter 24a constitute one laser sensor 23a, and the light emitter 24n and the light receiver 26n facing the light emitter 24n constitute one laser sensor 23n. In other words, the laser sensors 23a to 23n are disposed along the positions corresponding to the central opening 19 in the vertical direction.

The laser sensor 23 can detect an object positioned in the movement range of the scanner 20 and changes a detection area depending on the installation position of a subject support pole 70 (hereinafter simply referred to as a pole 70) for supporting the subject P in a standing position during standing position imaging. The pole 70 is an example of a subject support member.

More specifically, the laser sensor 23 detects an object positioned under the scanner 20 from before the start of imaging of the subject P to the end of imaging. In the present embodiment, the "object" detected by the laser sensor 23 includes the subject P and "an object that may be erroneously determined to be the subject P", which will be described later. Specifically, the laser sensor 23 detects an object positioned between the light emitter 24 and the light receiver 26.

For example, when no object is present between the light emitter 24a and the light receiver 26a, the light receiver 26a receives the laser beam emitted by the light emitter 24a. When an object is present between the light emitter 24a and the light receiver 26a, the laser beam is blocked, and the light receiver 26a does not receive the laser beam emitted by the light emitter 24a.

The numbers of light emitters 24, light receivers 26, and laser sensors 23 are not particularly limited. The method for detecting an object by the laser sensor 23 may be known techniques and is not particularly limited.

In the example illustrated in FIG. 1, the light receiver 26 is embedded in the floor surface, and the surface of the light receiver 26 is flush with the floor surface. The installation form of the light receiver 26 is not limited to this.

Each of the laser sensors 23a to 23n can be individually switched between an enabled state and a disabled state under the control of the control apparatus 28. The laser sensor 23 in the disabled state does not detect any object, for example. A control function 51 may be configured not to stop the operation of the scanner 20 when the laser sensor 23 considered to be in the disabled state detects an object, for example. Therefore, the detection areas of the laser sensors 23a to 23n change depending on which laser sensor 23 out of the laser sensors 23a to 23n is enabled by the control apparatus 28. The processing of changing the detection area depending on the installation position of the pole 70 will be described later.

The control apparatus 28 includes, for example, processing circuitry including a processor, such as a central processing unit (CPU), and a drive mechanism including a motor and an actuator. The processing circuitry implements these functions by a hardware processor executing computer programs stored in a storage (storage circuitry), for example.

The hardware processor refers to circuitry, such as a central processing unit (CPU), a graphics processing unit (GPU), an application specific integrated circuit (ASIC), a programmable logic device (e.g., a simple programmable logic device (SPLD) or a complex programmable logic device (CPLD)), and a field programmable gate array (FPGA).

Instead of being stored in the storage, the computer programs may be incorporated directly in the circuitry of the hardware processor. In this case, the hardware processor reads and executes the computer programs incorporated in the circuitry to implement the functions.

The hardware processor is not necessarily configured as a single circuit. Alternatively, a plurality of pieces of independent circuitry may be combined as a single hardware processor and implement the functions. The storage may be a non-transitory (hardware) storage medium. Alternatively, a plurality of components may be integrated into a single hardware processor and implement the functions.

The control apparatus 28, for example, rotates the rotating frame 17, moves the scanner 20 by the scanner drive apparatus 22, and moves the couchtop 33 of the couch apparatus 30. The couchtop 33 serves as a couch when the subject P is placed thereon in a supine position.

To tilt the scanner 20, for example, the control apparatus 28 controls the tilt mechanism 106 of the scanner drive apparatus 22 to rotate the rotating frame 17 about an axis parallel to the Z-axis direction based on the inclination angle (tilt angle) input to an input interface 43.

The control apparatus 28 grasps the rotation angle of the rotating frame 17 by an output of a sensor or the like, which is not illustrated. The control apparatus 28 sequentially transmits the rotation angle of the rotating frame 17 to processing circuitry 50. The control apparatus 28 may be provided to the gantry apparatus 10 or the console apparatus 40.

The couch apparatus 30 is an apparatus on which the subject P to be imaged is placed and that moves and introduces it into the rotating frame 17 of the gantry apparatus 10. The couch apparatus 30 includes, for example, a base 31, a couch drive apparatus 32, the couchtop 33, and a support frame 34.

The base 31 includes a housing that supports the support frame 34 movably in the vertical direction (Y-axis direction).

The couch drive apparatus 32 includes a motor and an actuator. The couch drive apparatus 32 moves the couchtop 33 on which the subject P is placed along the support frame 34 in the longitudinal direction (Z-axis direction) of the couchtop 33.

The couchtop 33 is a plate-like member on which the subject P is placed. The couch drive apparatus 32 moves the couchtop 33 backward to insert it into the central opening 19. The couch drive apparatus 32 moves the couchtop 33 forward to pull it out from the scanner 20.

The couch drive apparatus 32 may move not only the couchtop 33 but also the support frame 34 in the longitudinal direction of the couchtop 33. By contrast, the gantry apparatus 10 may be movable in the Z-axis direction, and the movement of the gantry apparatus 10 may be controlled such that the rotating frame 17 comes around the subject P. Alternatively, both the gantry apparatus 10 and the couchtop 33 may be movable.

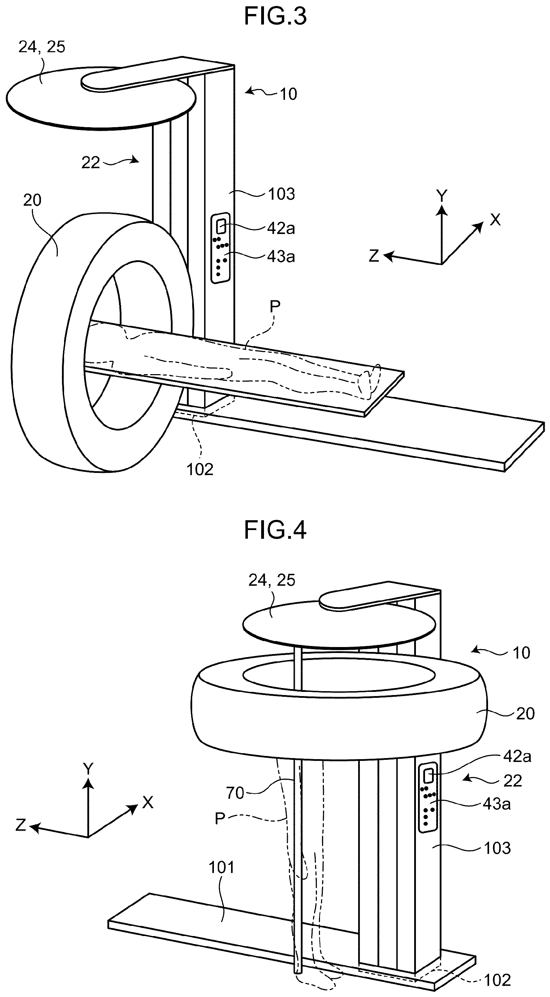

FIG. 3 is a perspective view of an example of the X-ray CT apparatus 1 that images the subject P in a supine position according to the first embodiment. FIG. 4 is a perspective view of an example of the X-ray CT apparatus 1 that images the subject P in a standing position according to the first embodiment.

The subject P placed on the couch apparatus 30 is imaged in a supine position as illustrated in FIG. 3. In the example in FIG. 3, the support column 103 and the scanner 20, which are positioned on the front side of the base 101 in FIG. 2, are moved to the rear side of the base 101 by the horizontal movement apparatus 102. Thus, the position of the support column 103 and the scanner 20 may be changed depending on the posture or the like of the subject P during imaging.

As illustrated in FIG. 4, the X-ray CT apparatus 1 can also image the subject P in a standing position. To image the subject P in a standing position, for example, the pole 70 is used to support the subject P in the standing position. The installation position of the pole 70 according to the present embodiment can be changed within the range of positions corresponding to the central opening 19.

As illustrated in FIGS. 3 and 4, the front surface of the support column 103 is provided with a display panel 42a and operation buttons 43a. With this configuration, for example, an operator can operate the operation buttons 43a while checking the contents displayed on the display panel 42a, thereby adjusting the position of the scanner 20 when imaging the subject P in a supine or standing position in an examination room where the X-ray CT apparatus 1 is installed.

The rear surface of the support column 103 may also be provided with a display panel and operation buttons similar to the display panel 42a and the operation buttons 43a. With this configuration, the operator can adjust the position of the scanner 20 without going around to the front side of the support column 103 when the operator checks the state of the subject P from the rear side of the support column 103 or when the front of the subject P faces in a different direction, such as the Z-axis direction.

The X-ray CT apparatus 1 according to the present embodiment can also image the subject P sitting in a chair or a wheelchair, that is, the subject P in a sitting position by the same method as that for imaging the subject P in a standing position illustrated in FIG. 4.

The gantry apparatus 10 and the couch apparatus 30 change the relative movement direction of the X-ray detector 15 provided to the scanner 20 and the subject P supported by the couchtop 33 by the horizontal movement apparatus 102, the slider 105, and the couch drive apparatus 32, for example.

To image the whole body of the subject P placed parallel to the Z-direction, for example, the gantry apparatus 10 and the couch apparatus 30 move the scanner 20 together with the support column 103 in the Z-direction by the horizontal movement apparatus 102.

To image the subject P along the orbitomeatal baseline (OM line) inclined about the X-axis from the Z-direction, for example, the gantry apparatus 10 and the couch apparatus 30 move the scanner 20 in the Z- and Y-directions by the horizontal movement apparatus 102 and the slider 105.

The console apparatus 40 includes, for example, a memory 41, a display 42, the input interface 43, and the processing circuitry 50. While the embodiment describes the console apparatus 40 as an apparatus separated from the gantry apparatus 10, the gantry apparatus 10 may include some or all of the components of the console apparatus 40.

The memory 41 is implemented by, for example, a semiconductor memory element, such as a random access memory (RAM) and a flash memory, a hard disk, or an optical disc. The memory 41 stores therein, for example, detection data, projection data, reconstructed image data, CT image data, or other data.

The data described above may be stored not in the memory 41 (or in addition to the memory 41) but in an external memory with which the X-ray CT apparatus 1 can communicate. The external memory is controlled by a cloud server managing the external memory and accepting read/write requests, for example.

The display 42 displays various kinds of information. The display 42 is an example of a display unit and a display medium. The display 42 is, for example, an output interface that displays medical images (CT images) generated by the processing circuitry and graphical user interface (GUI) images for receiving various operations performed by the operator, such as doctors and technicians.

Examples of the display 42 include, but are not limited to, a liquid crystal display, a cathode ray tube (CRT) display, an organic electroluminescence (EL) display, etc. The display 42 may be provided to the gantry apparatus 10. The display 42 may be a desktop display or a display apparatus (e.g., tablet terminal device) that can wirelessly communicate with the main body of the console apparatus 40.

The display panel 42a according to the present embodiment is included in the display 42.

The input interface 43 receives various input operations performed by the operator and outputs electrical signals indicating the contents of the received input operations to the processing circuitry 50.

The input interface 43, for example, receives input operations for inputting imaging conditions, including acquisition conditions for collecting detection data or projection data, reconstruction conditions for reconstructing CT images, and image processing conditions for generating post-processed images from the CT images.

The imaging conditions according to the present embodiment include posture information indicating the posture (e.g., supine, standing, or sitting position) of the subject P during imaging, and imaging information including information indicating the imaging site and information indicating whether to perform contrast-enhanced imaging.

The input interface 43 is implemented by, for example, a mouse, a keyboard, a touch panel, a trackball, a switch, a button, a joystick, a camera, an infrared sensor, a microphone, and other components. The input interface 43 may be implemented by a display apparatus (e.g., tablet terminal device) that can wirelessly communicate with the main body of the console apparatus 40.

The operation buttons 43a according to the present embodiment are included in the input interface 43.

In the present specification, the input interface is not limited only to those with physical operation components, such as a mouse and a keyboard. Examples of the input interface also include processing circuitry for electrical signals that receives electrical signals corresponding to an input operation from an external input device provided separately from the apparatus and outputs the electrical signals to the control circuitry.

The processing circuitry 50 controls the entire operations of the X-ray CT apparatus 1. The processing circuitry 50 includes, for example, the control function 51, a preprocessing function 52, a reconstruction processing function 53, an image processing function 54, a detection control function 55, and a detection function 56. The control function 51 is an example of an operation control unit and a display control unit. The detection control function 55 is an example of a detection control unit. The detection function 56 is an example of a detection unit.

The processing circuitry 50 implements these functions by a hardware processor executing computer programs stored in a storage (storage circuitry), for example.

The hardware processor refers to circuitry, such as a CPU, a GPU, an application specific integrated circuit, a programmable logic device or a complex programmable logic device, and a field programmable gate array. Instead of being stored in the storage, the computer programs may be incorporated directly in the circuitry of the hardware processor.

The hardware processor is not necessarily configured as a single circuit. Alternatively, a plurality of pieces of independent circuitry may be combined as a single hardware processor and implement the functions. The storage may be a non-transitory (hardware) storage medium. Alternatively, a plurality of components may be integrated into a single hardware processor and implement the functions.

The components of the console apparatus 40 or the processing circuitry 50 may be distributed and implemented by a plurality of pieces of hardware. The processing circuitry 50 is not necessarily included in the console apparatus 40 and may be implemented by a processing apparatus that can communicate with the console apparatus 40.

The processing apparatus is, for example, a workstation connected to one X-ray CT apparatus or an apparatus (e.g., cloud server) connected to a plurality of X-ray CT apparatuses and that collectively performs the processing equivalent to that performed by the processing circuitry 50 described below.

The control function 51 controls various functions of the processing circuitry 50 based on input operations received by the input interface 43. For example, the control function 51 controls the X-ray high voltage apparatus 14, the DAS 16, the control apparatus 28, and the couch drive apparatus 32 of the couch apparatus 30, thereby performing collection of detection data in the gantry apparatus 10.

When the detection function 56, which will be described later, detects that the subject P is present between the scanner 20 and the floor surface during imaging of the subject P, the control function 51 controls the scanner drive apparatus 22 to stop the operation of the scanner 20. This configuration can prevent the subject P from getting caught between the scanner 20 and the floor surface. The operation of the scanner 20 is, for example, a movement of the scanner 20 in the vertical direction and a tilt about the rotation axis of the scanner 20.

If the detection function 56 detects that the subject P is present between the scanner 20 and the floor surface when the operation of the scanner 20 is stopped, the control function 51 may perform control such that it does not receive an instruction by a user to move the scanner 20 toward the floor surface (in the opposite direction of the Y-axis direction).

The control function 51, for example, receives input of the imaging conditions. Specifically, the control function 51 receives input of the imaging conditions including the posture information and the imaging information.

The control function 51 may acquire the imaging conditions for the subject P by receiving an examination order from an external apparatus. For example, the control function 51 may receive an examination order including the imaging conditions for the subject P from a server of a radiology information system (RIS).

The control function 51, for example, controls the display 42 to display a GUI for operating the X-ray CT apparatus 1 and a display screen relating to the detection area. The display screen relating to the detection area will be described later.

The preprocessing function 52 performs preprocessing, such as logarithmic transformation, offset correction, sensitivity correction between channels, and beam hardening correction, on detection data output from the DAS 16 to generate projection data and stores the generated projection data in the memory 41.

The reconstruction processing function 53 performs reconstruction processing by the filtered back projection method, the iterative reconstruction method, or other methods on the projection data set by the preprocessing function 52 to generate CT image data and stores the generated CT image data in the memory 41.

The image processing function 54 converts CT image data into three-dimensional image data or cross-sectional image data of any desired section by known methods based on input operations received by the input interface 43. The conversion into three-dimensional image data may be performed by the preprocessing function 52.

The detection control function 55 changes the detection area of the laser sensor 23. The detection control function 55, for example, switches the laser sensor 23 between the enabled state and the disabled state depending on the imaging conditions. The following specifically describes a case where all the laser sensors 23 are in the disabled state in normal operation.

For example, when the posture information of the subject P received by the control function 51 indicates a supine position, the detection control function 55 maintains the disabled state of all the laser sensors 23. When the posture information of the subject P received by the control function 51 indicates a standing position, and the imaging information received by the control function 51 includes information indicating contrast-enhanced imaging, the detection control function 55 switches some of the laser sensors 23 to the enabled state depending on the installation position of the pole 70.

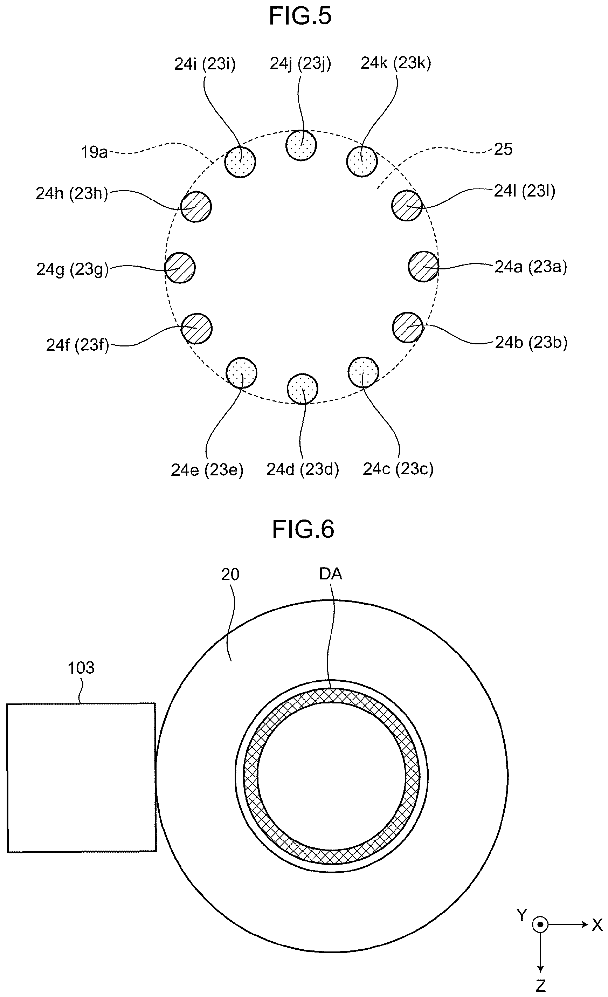

The following describes the processing for changing the detection area depending on the installation position of the pole 70 with reference to FIGS. 5 to 11. FIG. 5 is a top view of an example of the scanner 20 according to the first embodiment.

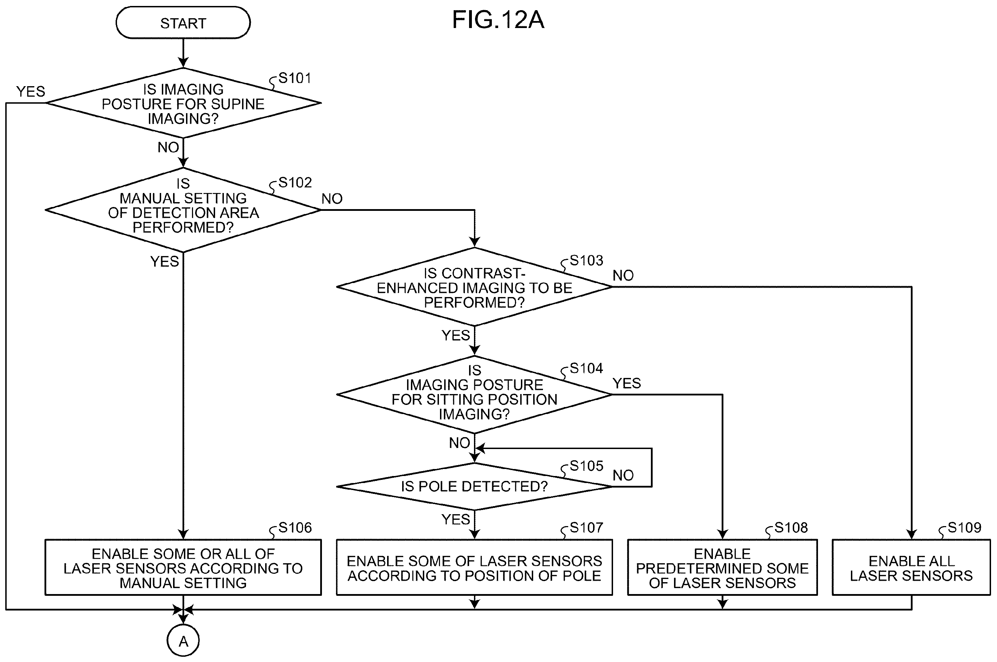

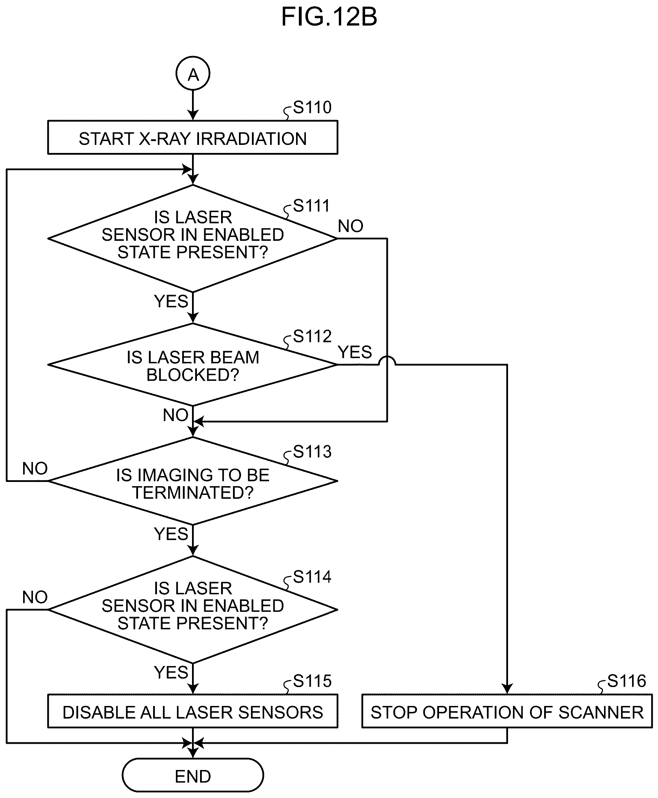

In the example illustrated in FIG. 5, 12 light emitters 24a to 241 are arranged in a circle to surround a circle 19a along the position (circle 19a) corresponding to the central opening 19 in the Y-axis direction of the scanner 20.

In FIG. 5, the upper part of the figure indicates the front direction of the gantry apparatus 10, and the lower part indicates the rear direction of the gantry apparatus 10. The number and installation positions of the light emitters 24 illustrated in FIG. 5 are given by way of example only, and the present embodiment is not limited thereto.

Although not illustrated in FIG. 5, 12 light receivers 26 corresponding to the respective light emitters 24a to 241 are provided on the floor surface. In other words, 12 laser sensors 23a to 231 are arranged in a circle to surround the central opening 19 in the vertical direction of the floor surface along the position corresponding to the central opening 19 in the vertical direction of the floor surface.

First, the following describes a case in which standing or sitting position imaging is performed and contrast-enhanced imaging is not performed. In this case, the detection control function 55 enables all the 12 laser sensors 23a to 231. FIG. 6 is a top view of an example of a detection area DA when contrast-enhanced imaging is not performed according to the first embodiment. As illustrated in FIG. 6, when contrast-enhanced imaging is not performed, the detection area DA is a cylindrical spatial area along the central opening 19 in the space between the scanner 20 and the floor surface.

Next, the following describes a case in which standing or sitting position imaging and contrast-enhanced imaging are performed. In this case, the detection control function 55 enables some of the 12 laser sensors 23a to 231 according to the installation position of the pole 70.

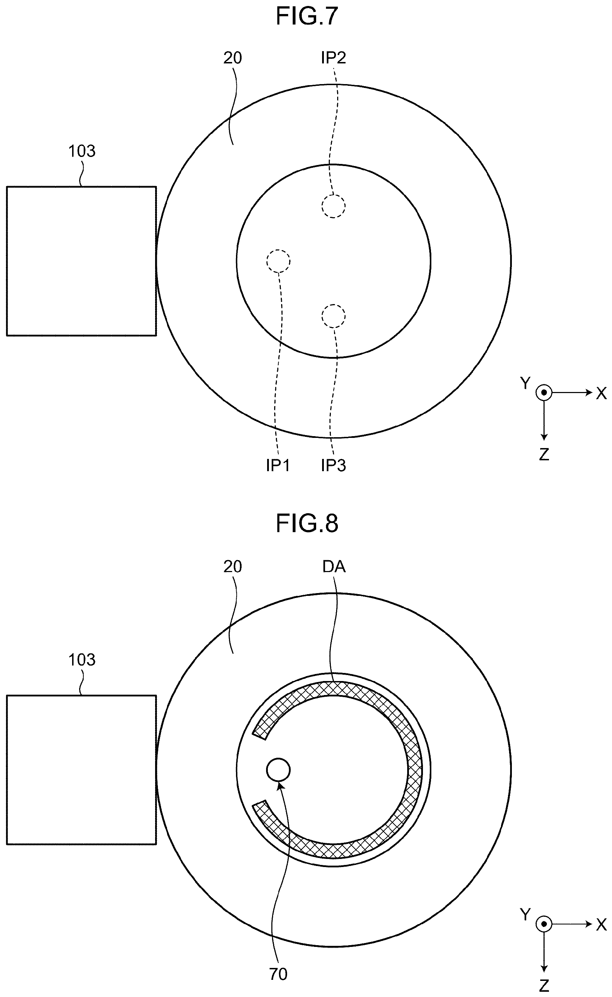

FIG. 7 is a view of an example of the installation positions of the pole 70 according to the first embodiment. In the example in FIG. 7, the pole 70 is installed at any one of a first installation position IP1 to a third installation position IP3. While the positions where the pole 70 according to the present embodiment can be installed are three positions of the first installation position IP1 to the third installation position IP3, these are given by way of example only. The positions where the pole 70 can be installed are not limited to these three positions.

The detection control function 55 identifies the position where the pole 70 is installed out of the first installation position IP1 to the third installation position IP3. The method for identifying the installation position of the pole 70 may be known techniques as appropriate.

For example, a camera is provided at a position where it can image the inside of the central opening 19 to identify the installation position of the pole 70 based on the image captured by the camera. Alternatively, contact sensors, mechanical switches, or the like may be provided at the first installation position IP1 to the third installation position IP3 to identify the installation position of the pole 70.

The detection control function 55 refers to correspondence information stored in the memory 41 or other components to identify the laser sensor 23 to be disabled out of the laser sensors 23a to 231. In the correspondence information, each of the first installation position IP1 to the third installation position IP3 is associated with information indicating the laser sensor 23 to be disabled.

In the correspondence information according to the present embodiment, the information identifying the laser sensor 23 to be disabled indicates the laser sensor 23 present in a direction in which the movement of the subject P is restricted out of the laser sensors 23a to 231.

The direction in which the movement of the subject P is restricted is specifically a direction in which the pole 70 is installed with respect to the subject P. The area in which the movement of the subject P is restricted is an area positioned in the direction in which the movement of the subject P is restricted, for example. This is because the subject P is standing with their back to the pole 70 and is unlikely to move in the direction of the pole 70.

In the correspondence information according to the present embodiment, the first installation position IP1 is associated with information identifying the laser sensor 23g, the second installation position IP2 is associated with information identifying the laser sensor 23j, and the third installation position IP3 is associated with information identifying the laser sensor 23d.

The laser sensor 23g is the laser sensor 23 closest to the first installation position IP1. Similarly, the laser sensor 23j is the laser sensor 23 closest to the second installation position IP2. Similarly, the laser sensor 23d is the laser sensor 23 closest to the third installation position IP3.

In other words, the correspondence information according to the present embodiment defines the laser sensor 23 closest to the installation position (the first installation position IP1 to the third installation position IP3) of the pole 70 as the laser sensor 23 present in the direction in which the movement of the subject P is restricted. The correspondence information defines the laser sensor 23 closest to the installation position (the first installation position IP1 to the third installation position IP3) of the pole 70 as the laser sensor 23 present in the area in which the movement of the subject P is restricted.

In the correspondence information according to the present embodiment, information identifying one laser sensor 23 is associated with one installation position. Alternatively, information identifying two or more laser sensors 23 may be associated with one installation position.

When it is identified that the pole 70 is installed at the first installation position IP1, for example, the detection control function 55 refers to the correspondence information and identifies the laser sensor 23g as the laser sensor 23 to be disabled based on the information identifying the laser sensor 23g associated with the first installation position IP1. The detection control function 55 enables the laser sensors 23 other than the identified laser sensor 23g.

FIG. 8 is a top view of an example of the detection area DA when the pole 70 is installed at the first installation position IP1 according to the first embodiment. As illustrated in FIG. 8, when the pole 70 is installed at the first installation position IP1, the detection area DA is an area obtained by excluding the detection area of the laser sensor 23g as a non-detection area from the cylindrical spatial area along the central opening 19 in the space between the scanner 20 and the floor surface.

In other words, in this case, the detection areas of the laser sensors 23 other than the laser sensor 23g are an example of a detection area of a detector. The detection area of the laser sensor 23g is an example of a non-detection area of the detector.

When it is identified that the pole 70 is installed at the second installation position IP2, for example, the detection control function 55 refers to the correspondence information and identifies the laser sensor 23j as the laser sensor 23 to be disabled based on the information identifying the laser sensor 23j associated with the second installation position IP2. The detection control function 55 enables the laser sensors 23 other than the identified laser sensor 23j.

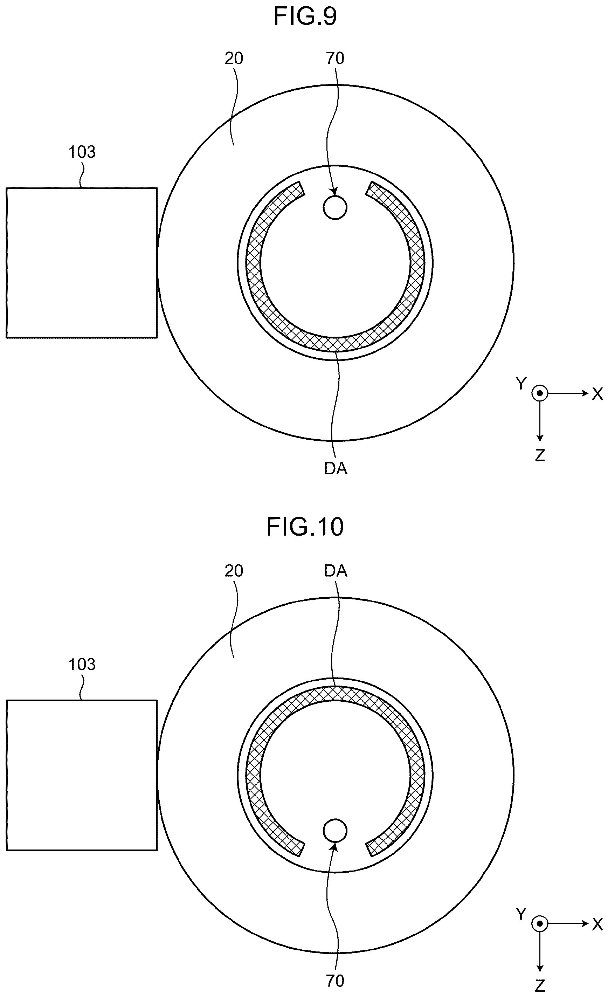

FIG. 9 is a top view of an example of the detection area DA when the pole 70 is installed at the second installation position IP2 according to the first embodiment. As illustrated in FIG. 9, when the pole 70 is installed at the second installation position IP2, the detection area DA is an area obtained by excluding the detection area of the laser sensor 23j as the non-detection area from the cylindrical spatial area along the central opening 19 in the space between the scanner 20 and the floor surface.

In other words, in this case, the detection areas of the laser sensors 23 other than the laser sensor 23j are an example of the detection area of the detector. The detection area of the laser sensor 23j is an example of the non-detection area of the detector.

When it is identified that the pole 70 is installed at the third installation position IP3, for example, the detection control function 55 refers to the correspondence information and identifies the laser sensor 23d as the laser sensor 23 to be disabled based on the information identifying the laser sensor 23d associated with the third installation position IP3. The detection control function 55 enables the laser sensors 23 other than the identified laser sensor 23d.

FIG. 10 is a top view of an example of the detection area DA when the pole 70 is installed at the third installation position IP3 according to the first embodiment. As illustrated in FIG. 10, when the pole 70 is installed at the third installation position IP3, the detection area DA is an area obtained by excluding the detection area of the laser sensor 23d as the non-detection area from the cylindrical spatial area along the central opening 19 in the space between the scanner 20 and the floor surface.

In other words, in this case, the detection areas of the laser sensors 23 other than the laser sensor 23d are an example of the detection area of the detector. The detection area of the laser sensor 23d is an example of the non-detection area of the detector.

When the posture information of the subject P received by the control function 51 indicates sitting position imaging, and the imaging information received by the control function 51 includes information indicating contrast-enhanced imaging, the detection control function 55 switches predetermined some of the laser sensors 23 to the enabled state. When the posture information of the subject P indicates sitting position imaging, and the imaging information includes information indicating contrast-enhanced imaging, the detection control function 55 according to the present embodiment switches the laser sensors 23 other than the laser sensor 23g to the enabled state.

In other words, in this case, the detection areas of the laser sensors 23 other than the laser sensor 23g are an example of the detection area of the detector. The detection area of the laser sensor 23g is an example of the non-detection area of the detector.

When the posture information of the subject P indicates sitting position imaging, and the imaging information includes information indicating contrast-enhanced imaging, the operator may be able to set which of the laser sensors 23 is to be switched to the enabled state.

For example, the detection control function 55 changes the detection areas of the laser sensors 23 according to the operator's instructions. In the following description, changing at least one of the detection area and the non-detection area of the laser sensor 23 according to the operator's instructions is also referred to as manual setting of the detection area DA.

Specifically, the detection control function 55 receives an input indicating whether to enable or disable each of the 12 laser sensors 23a to 231 from the operator via a detection area setting screen (not illustrated) displayed on the display panel 42a or other components. The detection control function 55 changes the detection areas of the laser sensors 23 according to the received input.

For example, when the detection control function 55 receives an input of an instruction to disable the laser sensors 23a and 23b and enable the other laser sensors 23 from the operator, it disables the laser sensors 23a and 23b and enables the other laser sensors 23.

In this case, the detection areas of the laser sensors 23 other than the laser sensors 23a and 23b are an example of the detection area of the detector. The detection areas of the laser sensors 23a and 23b are an example of the non-detection area of the detector.

By allowing the manual setting of the detection area DA, the operator can define a position convenient for passing a tube through the central opening 19 as the non-detection area and exclude it from the detection area DA, for example, when the operator desires to perform contrast-enhanced imaging of the subject P sitting in a wheelchair in a direction different from usual.

When the manual setting of the detection area DA is performed, the detection control function 55 according to the present embodiment does not perform the processing for changing the detection areas of the laser sensors 23 depending on the installation position of the pole 70 described above. In other words, in the present embodiment, the manual setting of the detection area DA takes priority over the processing for changing the detection areas of the laser sensors 23 depending on the installation position of the pole 70.

After the imaging of the subject P is finished, for example, the detection control function 55 disables all the laser sensors 23. This configuration prevents the laser beams from being continuously emitted from the light emitters 24 when the imaging of the subject P is not being performed, thereby reducing power consumption.

After the detection areas of the laser sensors 23 are changed by the detection control function 55, the control function 51 controls the display 42 (e.g., display panel 42a) to display a display screen for notifying the operator of the detection area DA.

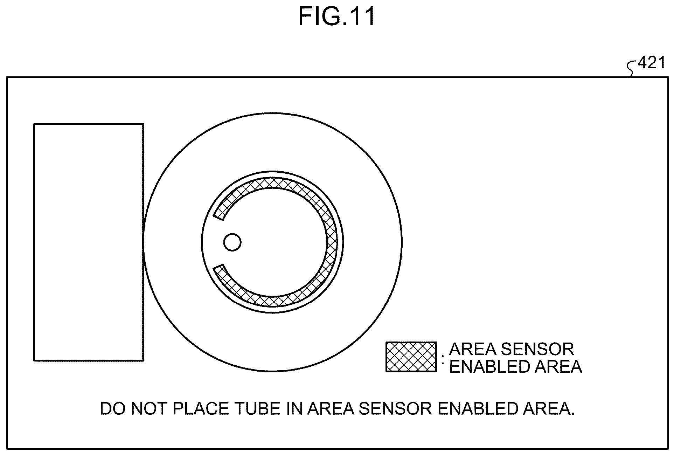

FIG. 11 is a view of an example of a display screen 421 for notifying the operator of the detection area DA according to the first embodiment. As illustrated in FIG. 11, the display screen 421 displays the detection area DA as an area sensor enabled area. The display screen 421 also displays a message for urging the operator not to place an injector tube (hereinafter simply referred to as a tube) used for contrast-enhanced imaging in the detection area DA (area sensor enabled area).

With this configuration, the operator can adjust the position of the tube so as not to place the tube in the detection area DA before starting the imaging of the subject P. By preventing the tube from being placed in the detection area DA, the X-ray CT apparatus 1 according to the first embodiment can prevent the operation of the scanner 20 from being stopped due to the tube erroneously determined to be the subject P. While the injector tube used for contrast-enhanced imaging is described as an example of an object that may be erroneously determined to be the subject P, the object that may be erroneously determined to be the subject P is not limited thereto. For example, the object that may be erroneously determined to be the subject P may be a tube connected to an oxygen mask, a tube connected to an intravenous infusion pack, or a cable connected to an electrode pad for measuring an electrocardiogram.

While the above has described an example where the display 42 is controlled to display a display screen for notifying the operator of the detection area DA, the method of notification is not limited thereto. For example, an indicator (not illustrated) composed of a plurality of LEDs may be provided on the surface of the exterior of the scanner 20, and the indicator may be used to provide notification.

For example, a plurality of LEDs may be provided on the periphery of the central opening 19 on the surface of the exterior of the cover 18, and a plurality of LEDs corresponding to the part indicating the detection area DA (area sensor enabled area) illustrated in FIG. 11 may be caused to emit light to provide notification. The exterior of the cover 18 is an example of the display medium. Alternatively, the LEDs may be provided on the surface of the outer peripheral portion of the cover 18. Still alternatively, the LEDs may be provided on the side surface (surface not having the central opening 19) of the scanner 20.

When the display 42 displays the display screen for notifying the operator of the detection area DA, the operator may need to move their line of sight or body to a position where they can visually recognize the display 42 to check the detection area DA. Therefore, when the operator is performing work, such as adjusting the position of the tube, they may need to perform the adjustment while repeatedly moving their line of sight significantly or moving near the display 42 to check the detection area DA.

By contrast, by providing a plurality of LEDs on the periphery of the central opening 19 on the surface of the exterior of the cover 18 as described above, the operator can adjust the position of the tube without moving their line of sight significantly or moving near the display 42. In other words, this configuration can improve the work efficiency of the operator engaged in examinations.

Alternatively, a projector (not illustrated) may be provided, for example. In this case, information indicating the detection area DA (area sensor enabled area) illustrated in FIG. 11 may be projected onto the exterior of the cover 18 or the floor surface. The floor surface in this case is an example of the display medium. Also in this case, the work efficiency of the operator engaged in examinations can be improved similarly to the example described above.

Still alternatively, a display apparatus (not illustrated), such as a liquid crystal display or an organic electroluminescence display, may be embedded in the floor surface to display the display screen illustrated in FIG. 11, for example. In this case, the display apparatus is preferably embedded at a position where the operator frequently performs work. Also in this case, the work efficiency of the operator engaged in examinations can be improved similarly to the example described above.

The display screen 421 may display the non-detection area as an area sensor disabled area and a message for urging the operator to place the injector tube used for contrast-enhanced imaging in the area sensor disabled area. With this configuration, the operator can adjust the position of the tube so as to place the tube in the non-detection area before starting the imaging of the subject P. By preventing the tube from being placed in the detection area DA, the X-ray CT apparatus 1 according to the first embodiment can prevent the operation of the scanner 20 from being stopped due to the tube erroneously determined to be the subject P.

The detection function 56 detects that the subject P is present between the scanner 20 and the floor surface. For example, when the laser beam emitted from the light emitter 24 of the laser sensor 23 in the enabled state is blocked, the detection function 56 detects that the subject P is present between the scanner 20 and the floor surface.

Specifically, when at least one of the light receivers 26 of the laser sensors 23 switched to the enabled state by the detection control function 55 fails to receive the laser beam for a period exceeding a predetermined time, the detection function 56 detects that the laser beam emitted from the light emitter 24 is blocked.

Next, the processing performed by the X-ray CT apparatus 1 according to the present embodiment is described. FIGS. 12A and 12B are a flowchart of an example of the processing performed by the X-ray CT apparatus 1 according to the embodiment. It is assumed that the control function 51 receives an input of the imaging conditions from the operator.

First, the detection control function 55 determines whether the imaging posture of the subject P is for supine imaging (Step S101). For example, if the posture information received by the control function 51 indicates supine imaging, the detection control function 55 determines that the imaging posture of the subject P is for supine imaging. By contrast, if the posture information received by the control function 51 indicates standing or sitting position imaging, the detection control function 55 determines that the imaging posture of the subject P is not for supine imaging.

If the imaging posture is for supine imaging (Yes at Step S101), the process proceeds to Step S110, which will be described later. By contrast, if the imaging posture is not for supine imaging (No at Step S101), the detection control function 55 determines whether manual setting of the detection area DA is performed (Step S102). For example, if the detection control function 55 receives an input indicating manual setting of the detection area DA from the operator via the setting screen for the detection area DA displayed on the display panel 42a, it determines that manual setting of the detection area DA is performed.

If manual setting of the detection area DA is performed (Yes at Step S102), the detection control function 55 enables some or all of the laser sensors 23 according to the contents of the manual setting of the detection area DA (Step S106), and the process proceeds to Step S110, which will be described later.

When the laser sensor 23 is enabled, the light emitter 24 starts to emit the laser beam toward the floor surface. If no object is present between the light emitter 24 and the light receiver 26, the laser beam is received by the light receiver 26.

By contrast, if manual setting of the detection area DA is not performed (No at Step S102), the detection control function 55 determines whether to perform contrast-enhanced imaging (Step S103). For example, if the imaging information received by the control function 51 includes information indicating contrast-enhanced imaging, the detection control function 55 determines that contrast-enhanced imaging is to be performed.

If contrast-enhanced imaging is not to be performed (No at Step S103), the detection control function 55 enables all the laser sensors 23 (Step S109), and the process proceeds to Step S110, which will be described later. By contrast, when contrast-enhanced imaging is to be performed (Yes at Step S103), the detection control function 55 determines whether the imaging posture of the subject P is for sitting position imaging (Step S104).

For example, if the posture information received by the control function 51 indicates sitting position imaging, the detection control function 55 determines that the imaging posture of the subject P is for sitting position imaging. By contrast, if the posture information received by the control function 51 indicates standing position imaging, the detection control function 55 determines that the imaging posture of the subject P is not for sitting position imaging.

If the imaging posture is for sitting position imaging (Yes at Step S104), the detection control function 55 enables predetermined some of the laser sensors 23 according to the contents of the manual setting of the detection area DA (Step S108), and the process proceeds to Step S110, which will be described later. By contrast, if the imaging posture is not for sitting position imaging (No at Step S104), the detection control function 55 determines whether the pole 70 is detected (Step S105).

For example, the detection control function 55 acquires an image captured by a camera provided at a position where it can image the inside of the central opening 19. If the pole 70 is detected in the captured image by a known image recognition technology, the detection control function 55 determines that the pole 70 is detected. If the pole 70 is not detected (No at Step S105), the detection control function 55 repeatedly performs the processing at Step S105.

If the pole 70 fails to be detected after a predetermined period of time has elapsed since the start of the processing of detecting the pole 70, the control function 51 may display a message for urging the operator to check whether the pole 70 is properly installed on the display panel 42a or other components.

By contrast, if the pole 70 is detected (Yes at Step S105), the detection control function 55 enables some of the laser sensors 23 according to the installation position of the pole (Step S107).

For example, the detection control function 55 analyzes the captured image in which the pole 70 is detected using a known image recognition technology to identify which of the first installation position IP1 to the third installation position IP3 in FIG. 7 the pole 70 is installed at. The detection control function 55 refers to the correspondence information stored in the memory 41 or other components and enables the laser sensors 23 other than the laser sensor 23 associated with the identified installation position.

Subsequently, the control function 51 operates the scanner 20 and starts to irradiate the subject P with X-rays (X-ray CT imaging of the subject P) (Step S110). Subsequently, the detection function 56 determines whether at least one of the laser sensors 23 is in the enabled state (Step S111).

For example, if the laser sensor 23 is enabled at any one of Steps S106 to S109, the detection function 56 determines that at least one of the laser sensors 23 is in the enabled state.

If the laser sensor 23 is not in the enabled state (No at Step S111), the process proceeds to Step S113, which will be described later. By contrast, if the laser sensor 23 is in the enabled state (Yes at Step S111), the detection function 56 determines whether the laser beam is blocked in at least one of the laser sensors 23 in the enabled state (Step S112).

For example, if the laser beam fails to be received by at least one of the light receivers 26 of the laser sensors 23 in the enabled state for a period exceeding a predetermined time, the detection function 56 determines that the laser beam is blocked in at least one of the laser sensors 23 in the enabled state.

If the laser beam is blocked (Yes at Step S112), the control function 51 stops the operation of the scanner 20 (Step S116), and the present processing is terminated. By contrast, if the laser beam is not blocked (No at Step S112), the detection control function 55 determines whether an instruction to terminate the imaging is received from the operator (Step S113). If an instruction to terminate the imaging is not received (No at Step S113), the process returns to Step S111.

By contrast, if an instruction to terminate the imaging is received (Yes at Step S113), the detection function 56 determines whether at least one of the laser sensors 23 is in the enabled state (Step S114) as in the processing at Step S111. If the laser sensor 23 is not in the enabled state (No at Step S114), the present processing is terminated.

By contrast, if the laser sensor 23 is in the enabled state (Yes at Step S114), the detection control function 55 disables all the laser sensors 23 (Step S115), and the present processing is terminated.

As described above, the X-ray CT apparatus 1 according to the first embodiment performs control to define at least part of the area between the outer periphery of the central opening 19 and the subject P in the X-Z plane as the detection area DA and detect that an object is present in the detection area DA and to define the area in which the movement of the subject is restricted during imaging in the X-Z plane as the non-detection area and exclude it from the detection area DA. When it is detected that an object is present in the detection area DA while the scanner 20 is operating, the X-ray CT apparatus 1 stops the operation of the scanner 20.

Typically, to perform contrast-enhanced imaging of a subject with an X-ray CT apparatus, an injector tube is passed through the inside of an opening of a scanner of the X-ray CT apparatus from the outside of the opening. In other words, the tube is present between the scanner and the floor surface in this case. Therefore, when an optical sensor is used to detect that the subject is present between the scanner and the floor surface, for example, light emitted from the light emitter of the optical sensor may be blocked by the tube, resulting in erroneous determination that the subject is present between the scanner and the floor surface. Such erroneous determination may cause the scanner to stop during the imaging of the subject. If the scanner stops during the imaging of the subject, the imaging is likely to need to be performed again, which may lead to unnecessary exposure of the subject.

By contrast, the X-ray CT apparatus 1 according to the present embodiment performs control to define the area in which the movement of the subject is restricted during imaging in the X-Z plane as the non-detection area and exclude it from the detection area DA as described above. If an object is present between the scanner 20 and the floor surface in the area in which the movement of the subject is restricted during imaging in the X-Z plane, the operation of the scanner 20 is not stopped. Therefore, by placing the tube in this area, the operation of the scanner 20 is not stopped if the tube is present between the scanner 20 and the floor surface. In other words, the X-ray CT apparatus 1 according to the first embodiment can reduce the possibility of erroneously determining an object that may be present between the scanner 20 and the floor surface as the subject P and causing the scanner 20 to stop operating during imaging of the subject P.

### Second Embodiment

The first embodiment above has described an aspect that reduces the possibility of erroneously determining an object that is not the subject P as the subject P by changing the detection area DA of the laser sensors 23. A second embodiment describes an aspect that reduces the possibility of erroneously determining an object that is not the subject P as the subject P based on the results of detection by the laser sensors 23.

The following mainly describes the points different from those according to the embodiment above, and detailed explanation of the points common to the already described contents is omitted. The embodiments described below may be implemented individually or in combination as appropriate.

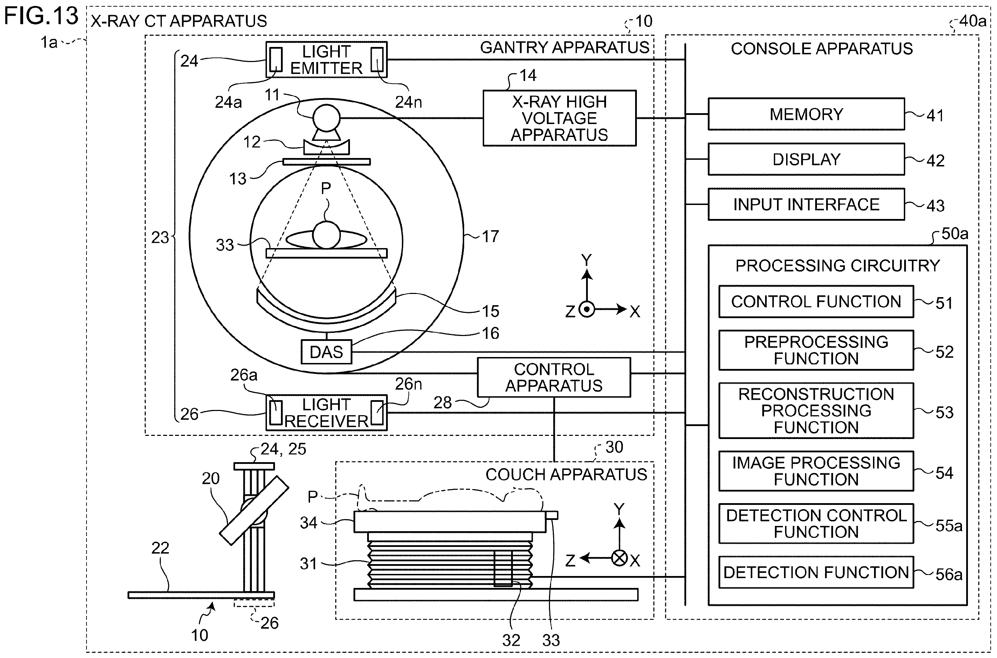

First, the configuration of an X-ray CT apparatus 1a according to the second embodiment is described. FIG. 13 is a block diagram of an example of the configuration of the X-ray CT apparatus 1a according to the second embodiment. The X-ray CT apparatus 1a according to the second embodiment has substantially the same configuration as the X-ray CT apparatus 1 in FIG. 1 but differs from the X-ray CT apparatus 1 in FIG. 1 in that it includes a console apparatus 40a.

The console apparatus 40a has substantially the same configuration as the console apparatus 40 according to the first embodiment in FIG. 1 but differs from the console apparatus 40 in FIG. 1 in that it includes processing circuitry 50a. The processing circuitry 50a has substantially the same configuration as the processing circuitry 50 in FIG. 1 but differs from the processing circuitry 50 in FIG. 1 in that it includes a detection control function 55a and a detection function 56a.

When the imaging posture of the subject P is for standing or sitting position imaging, the detection control function 55a enables all the laser sensors 23. For example, if the posture information of the subject P received by the control function 51 indicates standing or sitting position imaging, the detection control function 55a switches all the laser sensors 23 to the enabled state.

The detection function 56a detects the subject P and an object other than the subject P in the detection area DA. For example, the detection function 56a determines whether the subject P or an object other than the subject P is detected depending on the number of laser sensors 23 that determine that an adjacent laser beam is blocked.

If there are a plurality of laser sensors 23 that determine that the laser beam is blocked, but there is no laser sensor that determines that the adjacent laser beam is blocked, the number of laser sensors 23 that determine that the adjacent laser beam is blocked is one.

Specifically, if the number of laser sensors 23 that determine that the adjacent laser beam is blocked is equal to or larger than a threshold, the detection function 56a determines that the subject P is detected in the detection area DA. By contrast, if the number of laser sensors 23 that determine that the adjacent laser beam is blocked is smaller than the threshold, the detection function 56a determines that an object other than the subject P is detected in the detection area DA.

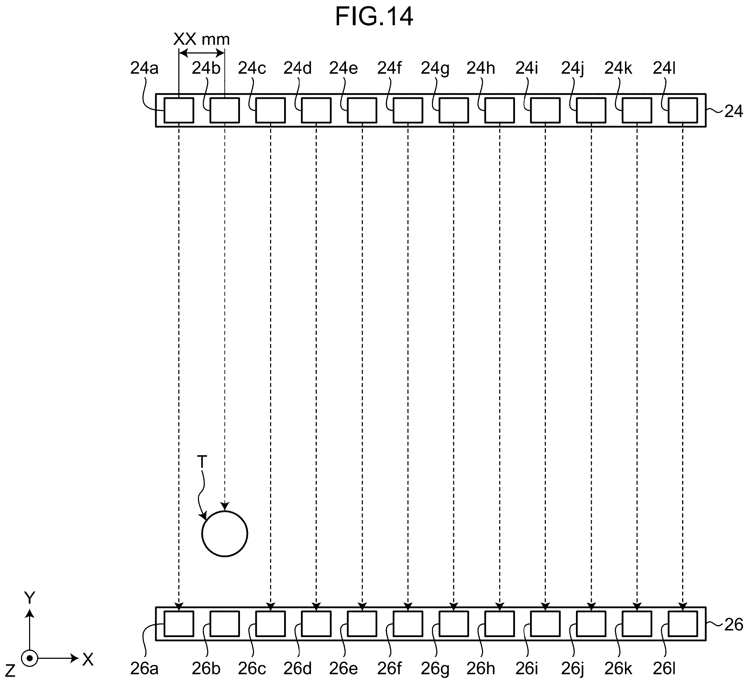

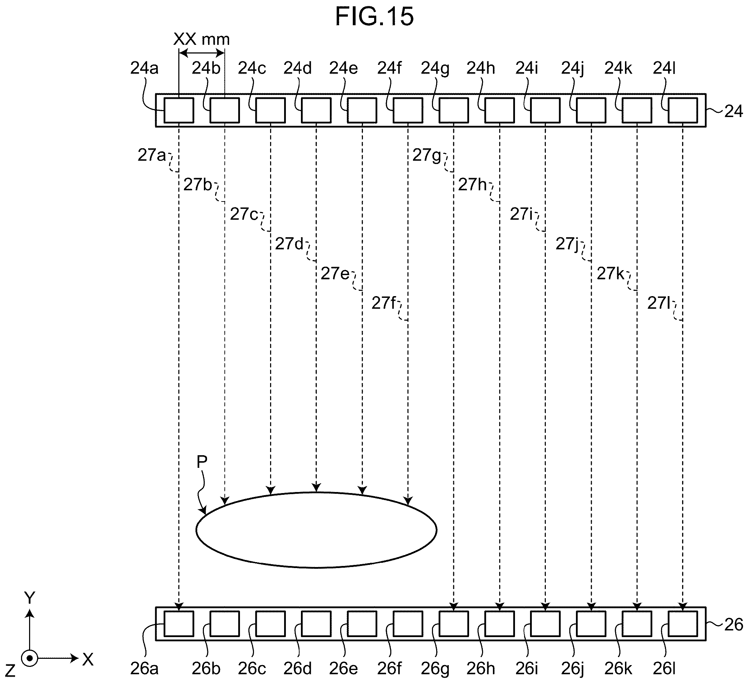

FIGS. 14 and 15 are diagrams for explaining an example of the processing for determining whether the subject P or an object other than the subject P is detected according to the second embodiment.

FIGS. 14 and 15 are developed views of the light emitters 24a to 241 and the light receivers 26a to 261 arranged in a circle. In the examples in FIGS. 14 and 15, the light emitters 24a to 241 emit laser beams 27a to 271 to the light receivers 26a to 261, respectively.

FIG. 14 illustrates an example of a case where an object other than the subject P is determined to be detected in the detection area DA. FIG. 15 illustrates an example of a case where the subject P is determined to be detected in the detection area DA. In FIGS. 14 and 15, the threshold of the number of laser sensors 23 that determine that the adjacent laser beam is blocked is two.

In the example in FIG. 14, a tube T blocks the laser beam 27b emitted from the light emitter 24b. In this case, only the laser sensor 23b determines that the laser beam is blocked, so the detection function 56a determines that the number of laser sensors 23 that determine that the adjacent laser beam is blocked is one. The number of laser sensors 23 that determine that the adjacent laser beam is blocked is smaller than the threshold, so the detection function 56a determines that an object other than the subject P is detected in the detection area DA.

As illustrated in FIG. 14, when the interval between the laser sensors 23 is XX mm, and the number of laser sensors 23 that determine that the adjacent laser beam is blocked is one, it can be found out that the size of the object that blocks the laser beam is smaller than 2 × XX mm. This is because, when the size of the object is equal to or larger than 2 × XX mm, the number of laser sensors 23 that determine that the adjacent laser beam is blocked is always two or more.

In other words, in the examples in FIGS. 14 and 15, the detection function 56a determines that an object other than the subject P is detected in the detection area DA when the size of the object that blocks the laser beam can be determined to be smaller than 2 × XX mm.

In the example in FIG. 15, the arm of the subject P blocks the laser beams 27b to 27f emitted from the light emitters 24b to 24f, respectively. In this case, five laser sensors 23b to 23f determine that the laser beam is blocked, so the detection function 56a determines that the number of laser sensors 23 that determine that the adjacent laser beam is blocked is five. The number of laser sensors 23 that determine that the adjacent laser beam is blocked is equal to or larger than the threshold, so the detection function 56a determines that the subject P is detected in the detection area DA.

As illustrated in FIG. 15, when the interval between the laser sensors 23 is XX mm, and the number of laser sensors 23 that determine that the adjacent laser beam is blocked is two or more, it can be found out that the size of the object that blocks the laser beam is XX mm or more. This is because, when the size of the object is smaller than XX mm, the number of laser sensors 23 that determine that the adjacent laser beam is blocked is never two or more.

In other words, in the examples in FIGS. 14 and 15, the detection function 56a determines that the subject P is detected in the detection area DA when the size of the object that blocks the laser beam can be determined to be equal to or larger than XX mm.

The control function 51 controls the scanner drive apparatus 22 to stop the operation of the scanner 20 only when the detection function 56a determines that the subject P is detected in the detection area DA. In other words, the control function 51 does not stop the operation of the scanner 20 when the detection function 56a determines that an object other than the subject P is detected in the detection area DA.

With this configuration, the X-ray CT apparatus 1a according to the second embodiment can prevent the operation of the scanner 20 from being stopped due to an object, such as a tube, erroneously determined to be the subject P.

The detection function 56a may perform the processing for determining whether the subject P or an object other than the subject P is detected depending on the number of laser sensors 23 that determine that the adjacent laser beam is blocked only in contrast-enhanced imaging.

The control function 51 may notify the operator whether the position of the laser sensor 23 allows for blocking of the laser beam and whether an object other than the subject P is detected.

For example, the control function 51 may provide notification on the combination of the state (enabled or disabled) of the laser sensor 23 and the state (present or absent) of detection of an object other than the subject P. In this case, the control function 51 also detects an object other than the subject P outside the detection area DA (laser sensor 23 in the disabled state).

For convenience of explanation, the combination of the state (enabled/disabled) of the laser sensor 23 and the state (present/absent) of detection of an object other than the subject P is denoted as follows: "laser sensor 23: enabled/disabled, object detection: present/absent".

For example, the control function 51 may display, on the display 42, a display screen that displays "laser sensor 23: enabled, object detection: present", "laser sensor 23: enabled, object detection: absent", "laser sensor 23: disabled, object detection: present", and "laser sensor 23: disabled, object detection: absent" instead of the detection area DA (area sensor enabled area) illustrated in FIG. 11 described above.

With this configuration, for example, the operator who performs work, such as adjusting the position of the tube, can perform the work while checking whether the position of the laser sensor 23 allows for blocking of the laser beam and whether an object other than the subject P is detected.

The notification described above may be provided using an indicator, a projector, a display embedded in the floor surface, or the like similarly to the notification of the detection area DA according to the first embodiment.

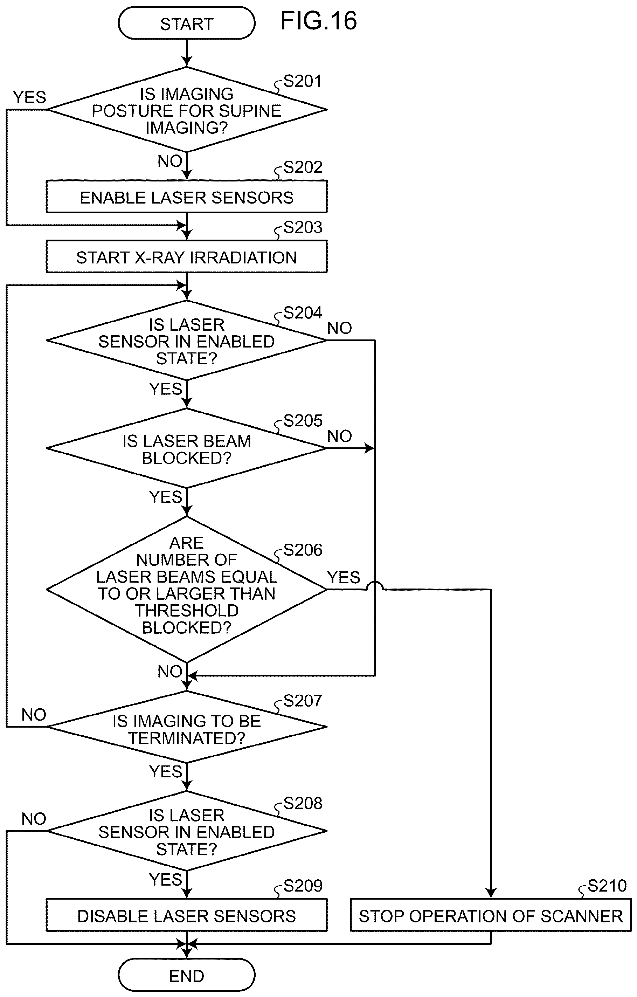

Next, the processing performed by the X-ray CT apparatus 1a according to the second embodiment is described. FIG. 16 is a flowchart of an example of the processing performed by the X-ray CT apparatus 1a according to the second embodiment. It is assumed that the control function 51 receives an input of the imaging conditions from the operator. The processing at Step S201 in FIG. 16 is the same as that at Step S101 in FIG. 12A, so explanation thereof is omitted.

If the imaging posture is for supine imaging (Yes at Step S201), the process proceeds to Step S203, which will be described later. By contrast, if the imaging posture is not supine imaging (No at Step S201), the detection control function 55a enables all the laser sensors 23 (Step S202). The processing at next Step S203 is the same as that at Step S110 in FIG. 12B, so explanation thereof is omitted.

After Step S203, the detection function 56a determines whether the laser sensor 23 is in the enabled state (Step S204). For example, if all the laser sensors 23 are enabled at Step S202, the detection function 56a determines that the laser sensor 23 is in the enabled state. If the laser sensor 23 is not in the enabled state (No at Step S204), the process proceeds to Step S207, which will be described later.

By contrast, if the laser sensor 23 is in the enabled state (Yes at Step S204), the detection function 56a determines whether the laser beam is blocked in at least one of the laser sensors 23 (Step S205) as in the processing at Step S112 in FIG. 12B. If the laser beam is not blocked (No at Step S205), the process proceeds to Step S207.

By contrast, if the laser beam is blocked (Yes at Step S205), the detection function 56a determines whether the number of laser sensors 23 that determine that the adjacent laser beam is blocked is equal to or larger than the threshold (Step S206).

If the number of laser sensors 23 that determine that the adjacent laser beam is blocked is equal to or larger than the threshold (Yes at Step S206), the control function 51 stops the operation of the scanner 20 (Step S210) as in the processing at Step S116 in FIG. 12B, and the present processing is terminated.

By contrast, if the number of laser sensors 23 that determine that the adjacent laser beam is blocked is smaller than the threshold (No at Step S206), the detection control function 55a determines whether an instruction to terminate the imaging is received from the operator (Step S207) as in the processing at Step S113 in FIG. 13. If an instruction to terminate the imaging is not received (No at Step S207), the process returns to Step S204.

By contrast, if an instruction to terminate the imaging is received (Yes at Step S207), the detection function 56a determines whether the laser sensor 23 is in the enabled state (Step S208) as in the processing at Step S204. If the laser sensor 23 is not in the enabled state (No at Step S208), the present processing is terminated.

By contrast, if the laser sensor 23 is in the enabled state (Yes at Step S208), the detection control function 55a disables all the laser sensors 23 (Step S209) as in the processing at Step S115 in FIG. 12B, and the present processing is terminated.

As described above, the X-ray CT apparatus 1a according to the second embodiment defines at least part of the area between the outer periphery of the central opening 19 and the subject P in the X-Z plane as the detection area DA and detects that the subject P and an object other than the subject P are present in the detection area DA. When it is detected that an object other than the subject P is present in the detection area DA while the scanner 20 is operating, the X-ray CT apparatus 1a stops the operation of the scanner 20.

With this configuration, the X-ray CT apparatus 1a according to the second embodiment can stop the operation of the scanner 20 only when it detects that the subject P is present in the detection area DA. Therefore, if an object other than the subject P, such as a tube, is present between the scanner 20 and the floor surface during imaging of the subject P, the operation of the scanner 20 is not stopped. In other words, the X-ray CT apparatus 1a according to the second embodiment can reduce the possibility of erroneously determining an object that may be present between the scanner 20 and the floor surface as the subject P and causing the scanner 20 to stop operating during imaging of the subject P.

The embodiment described above can also be implemented by modifying some of the components or functions of the X-ray CT apparatus 1 as appropriate. Therefore, the following describes modifications according to the embodiments above as other embodiments. The following mainly describes the points different from those according to the embodiments above, and detailed explanation of the points common to the already described contents is omitted. The modifications described below may be implemented individually or in combination as appropriate.

### First Modification

As described in the first embodiment, when the posture information of the subject P indicates sitting position imaging, and the imaging information includes information indicating contrast-enhanced imaging, the detection control function 55 enables predetermined some of the laser sensors 23.

The detection control function 55, however, may enable predetermined some of the laser sensors 23 also when the posture information of the subject P indicates standing position imaging and the imaging information includes information indicating contrast-enhanced imaging. The detection control function 55 may enable different laser sensors 23 between standing position imaging and sitting position imaging.

The present modification can reduce the possibility of erroneously determining an object that may be present between the scanner 20 and the floor surface as the subject P and causing the scanner 20 to stop operating during imaging without performing the processing of identifying the installation position of the pole 70 in the standing position imaging of the subject P.

### Second Modification

As described in the first embodiment, when the posture information of the subject P indicates standing position imaging, and the imaging information includes information indicating contrast-enhanced imaging, the detection control function 55 enables some of the laser sensors 23 according to the installation position of the pole 70.

In the present modification, the front direction is set in advance. When the imaging information includes information indicating contrast-enhanced imaging, the detection control function 55 enables some of the laser sensors 23 according to the front direction.

In the present modification, the control function 51 sets the front direction by receiving an input specifying the laser sensors 23a to 231 from the operator via a front setting screen (not illustrated) for setting the front direction displayed on the display 42. In the following description, setting the front direction is also referred to as front setting.

In the correspondence information according to the present modification, information identifying the laser sensor 23 indicating the front direction is associated with information identifying the laser sensor 23 to be disabled.

Specifically, the correspondence information according to the present modification associates the information identifying the laser sensor 23 indicating the front direction with the information identifying the laser sensor 23 farthest from the laser sensor 23 indicating the front direction.

More specifically, when the laser sensor 23 indicating the front direction is the laser sensor 23a, the information identifying the laser sensor 23a indicating the front direction is associated with the information identifying the laser sensor 23g serving as the information identifying the laser sensor 23 to be disabled in the correspondence information.

In the correspondence information, information identifying the laser sensor 23 indicating one front direction may be associated with information identifying two or more laser sensors 23 to be disabled.

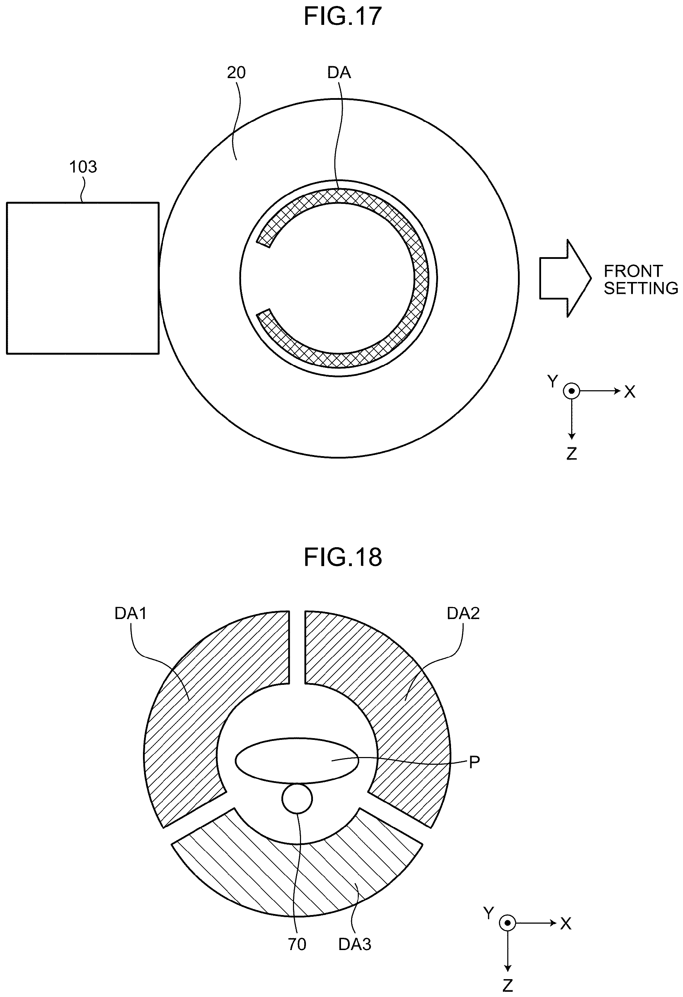

FIG. 17 is a top view of an example of the detection area DA determined according to the front setting according to a second modification. FIG. 17 illustrates the detection area DA when the front setting is performed to set the laser sensor 23a as the front.

In this example, the detection control function 55 refers to the correspondence information stored in the memory 41 or other components and identifies the laser sensor 23g as the laser sensor 23 to be disabled based on the information identifying the laser sensor 23g associated with the information identifying the laser sensor 23a set as the front. The detection control function 55 enables the laser sensors 23 other than the identified laser sensor 23g.

In other words, in this case, the detection areas of the laser sensors 23 other than the laser sensor 23g are an example of the detection area of the detector. The detection area of the laser sensor 23g is an example of the non-detection area of the detector.

The detection control function 55 according to the present modification may perform the processing of enabling some of the laser sensors 23 according to the installation position of the pole 70 as in the first embodiment only when the front setting is not performed.

According to the present modification, the operator can exclude any desired position from the detection area DA as the non-detection area when the pole 70 is not installed, such as when performing sitting position imaging of the subject P.

### Third Modification

As described in the first embodiment, when the posture information of the subject P indicates standing position imaging, and the imaging information includes information indicating contrast-enhanced imaging, the detection control function 55 enables some of the laser sensors 23 according to the installation position of the pole 70.

In the present modification, when the posture information of the subject P indicates sitting position imaging, and the imaging information includes information indicating contrast-enhanced imaging, the detection control function 55 enables some of the laser sensors 23 according to the installation position and orientation of a member for supporting the subject, such as a chair or a wheelchair used for imaging.

In the correspondence information according to the present modification, information indicating the installation position and orientation of the member (hereinafter referred to as a chair or the like) for supporting the subject, such as a chair or a wheelchair used for imaging, is associated with information identifying the laser sensor 23 to be disabled. The chair or the like is an example of the subject support member.

Specifically, the correspondence information according to the present modification associates the information indicating the installation position and orientation of the chair or the like with the information identifying the laser sensor 23 closest to a member for supporting the back of the subject P, such as the backrest of the chair or the like, when the chair or the like is placed at the installation position and orientation of the chair or the like.

In the correspondence information, the information indicating the installation position and orientation of the chair or the like may be associated with information identifying two or more laser sensors 23 to be disabled (e.g., the farthest 5% of the laser sensors 23 from the member for supporting the back of the subject P). In the correspondence information, the information indicating the installation position of the chair or the like may be associated with the information identifying the laser sensor 23 to be disabled.

When the posture information of the subject P indicates sitting position imaging, and the imaging information includes information indicating contrast-enhanced imaging, the detection control function 55 according to the present modification acquires an image captured by a camera provided at a position where it can image the inside of the central opening 19. Subsequently, the detection control function 55 identifies the installation position and orientation of the chair or the like by a known image recognition technology.

Subsequently, the detection control function 55 refers to the correspondence information stored in the memory 41 or other components and identifies the laser sensor 23 to be disabled based on the information identifying the laser sensor 23 to be disabled associated with the information indicating the identified installation position and orientation of the chair or the like. The detection control function 55 enables the laser sensors 23 other than the identified laser sensor 23.

In this case, the detection areas of the laser sensors 23 other than the laser sensor 23 identified as the laser sensor 23 to be disabled are an example of the detection area of the detector. The detection area of the laser sensor 23 identified as the laser sensor 23 to be disabled is an example of the non-detection area of the detector.

According to the present modification, the detection areas of the laser sensors 23 can be changed based on the position of the member for supporting the subject when sitting position imaging and contrast-enhanced imaging are performed. In other words, the present modification can reduce the possibility of erroneously determining an object that may be present between the scanner 20 and the floor surface as the subject P and causing the scanner 20 to stop operating during imaging of the subject P.

### Fourth Modification

As described above in the first embodiment, the detection control function 55 enables or disables the laser sensors 23a to 23l individually. In the present modification, the laser sensors 23a to 23l are divided into groups, and the detection control function 55 enables or disables them in units of groups.

The laser sensors 23 according to the present modification are grouped into a first area DA1, a second area DA2, and a third area DA3. The first area DA1, the second area DA2, and the third area DA3 are examples of a group area. Information defining the grouping of the laser sensors 23 is stored in the memory 41 or other components.

In the correspondence information according to the present modification, the installation position of the pole 70 is associated with an area to be disabled. Specifically, the correspondence information according to the present modification associates the installation position of the pole 70 with the area closest to the installation position.

With this configuration, when the posture information of the subject P indicates sitting position imaging, and the imaging information includes information indicating contrast-enhanced imaging, the detection control function 55 according to the present modification can refer to the correspondence information and enable the laser sensors 23 belonging to the areas other than the area closest to the installation position of the pole 70 out of the first area DA1 to the third area DA3.

FIG. 18 is a top view for explaining an example of the positional relation between the installation position of the pole 70 and the area to be enabled according to a fourth modification. In the example in FIG. 18, the area closest to the installation position of the pole 70 is the third area DA3. In other words, the installation position of the pole in FIG. 18 is associated with the third area DA3 in the correspondence information.

Therefore, in the example in FIG. 18, the detection control function 55 refers to the correspondence information and identifies the third area DA3 as the area to be disabled. Subsequently, the detection control function 55 enables the laser sensors 23 belonging to the first area DA1 and the second area DA2 other than the third area DA3.

To perform manual setting of the detection area, the detection control function 55 according to the present modification receives an input indicating whether to enable or disable each of the first area DA1 to the third area DA3 from the operator via the detection area setting screen (not illustrated) displayed on the display panel 42a or other components. The detection control function 55 changes the detection areas of the laser sensors 23 according to the received input.

For example, when the detection control function 55 receives an instruction to disable the first area DA1 and enable the other areas from the operator, it disables the laser sensors 23 belonging to the first area DA1 and enables the laser sensors 23 belonging to the second area DA2 and the third area DA3.

In other words, in this case, the detection areas of the laser sensors 23 belonging to the second area DA2 and the third area DA3 are an example of the detection area of the detector. The detection areas of the laser sensors 23 belonging to the first area DA1 are an example of the non-detection area of the detector.

When the posture information of the subject P indicates sitting position imaging, and the imaging information includes information indicating contrast-enhanced imaging, the detection control function 55 according to the present modification enables the laser sensors 23 belonging to a predetermined area out of the first area DA1 to the third area DA3.

According to the present modification, the detection areas of the laser sensors 23 are determined in units of groups (areas). This configuration facilitates the operator's visually grasping which area is the detection area and which is the non-detection area when the detection areas of the laser sensors 23 are displayed.

### Fifth Modification

As described above in the first embodiment, when the posture information of the subject P indicates standing position imaging, and the imaging information includes information indicating contrast-enhanced imaging, the detection control function 55 enables the laser sensors 23 other than the laser sensor 23 closest to the installation position of the pole 70 (the first installation position IP1 to the third installation position IP3).

In the present modification, when the posture information of the subject P indicates standing position imaging, and the imaging information includes information indicating contrast-enhanced imaging, the detection control function 55 enables the laser sensors 23 other than the laser sensors 23 present in the area behind the pole 70 as viewed from the subject P.

In the correspondence information according to the present modification, the installation position of the pole 70 is associated with information identifying a plurality of laser sensors 23 to be disabled. Specifically, the correspondence information according to the present modification associates the installation position of the pole 70 with the identification information of a plurality of laser sensors 23 present in the area behind the pole 70 as viewed from the subject P when the pole 70 is installed at the installation position.

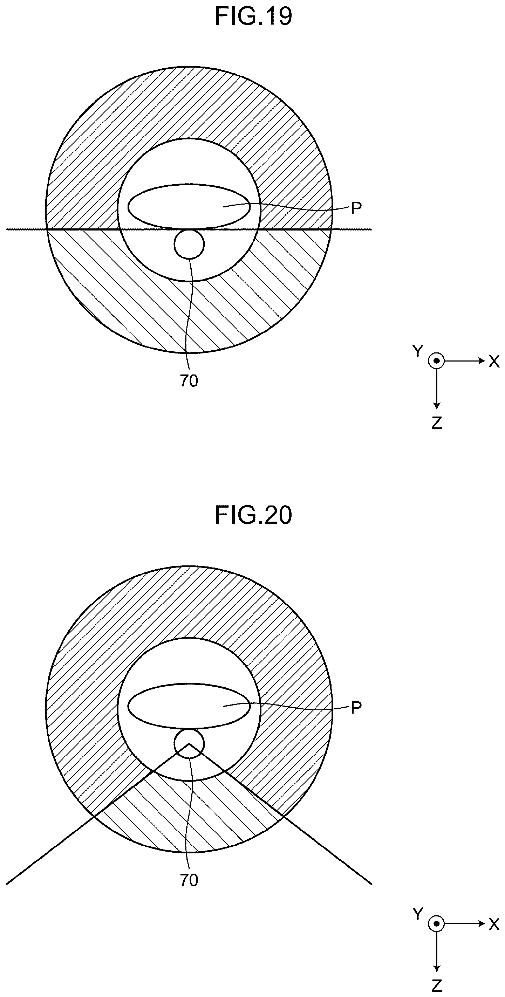

FIG. 19 is a top view for explaining an example of the positional relation between the installation position of the pole 70 and the laser sensors 23 to be enabled according to a fifth modification. In the example in FIG. 19, the area indicated by thick diagonal lines is the area behind the pole 70 as viewed from the subject P when the pole 70 is installed at the position illustrated in FIG. 19.

In other words, in the correspondence information in the example in FIG. 19, the installation position of the pole illustrated in FIG. 19 is associated with the identification information of the laser sensors 23 present in the area indicated by the thick diagonal lines.

Therefore, in the example in FIG. 19, the detection control function 55 refers to the correspondence information and identifies the laser sensors 23 present in the area indicated by the thick diagonal lines as the laser sensors 23 to be disabled. Subsequently, the detection control function 55 enables the laser sensors 23 belonging to the first area DA1 and the second area DA2 other than the identified laser sensors 23.

In this case, the detection areas of the laser sensors 23 other than the laser sensors 23 present in the area indicated by the thick diagonal lines in FIG. 19 are an example of the detection area of the detector. The detection areas of the laser sensors 23 present in the area indicated by the thick diagonal lines in FIG. 19 are an example of the non-detection area of the detector.

The present modification can increase the area where the tube can be placed compared with the first embodiment.

### Sixth Modification

As described in the fifth modification, when the posture information of the subject P indicates standing position imaging, and the imaging information includes information indicating contrast-enhanced imaging, the detection control function 55 enables the laser sensors 23 other than all the laser sensors 23 present in the area behind the pole 70 as viewed from the subject P.

In this case, however, the detection control function 55 may enable the laser sensors 23 other than some of the laser sensors 23 present in the area behind the pole 70 as viewed from the subject P.

FIG. 20 is a top view for explaining an example of the positional relation between the installation position of the pole 70 and the laser sensors 23 to be enabled according to a sixth modification. In the correspondence information in the example in FIG. 20, the installation position of the pole illustrated in FIG. 20 is associated with the identification information of a plurality of laser sensors 23 present in the area indicated by the thick diagonal lines.

Specifically, the correspondence information in the example in FIG. 20 associates the installation position of the pole 70 with the identification information of a plurality of laser sensors 23 present in the area 120° behind the pole 70 when the pole 70 is installed at the installation position and the area directly behind the pole 70 is 0° behind the pole 70.

In this case, the detection areas of the laser sensors 23 other than the laser sensors 23 present in the area indicated by the thick diagonal lines in FIG. 20 are an example of the detection area of the detector. The detection areas of the laser sensors 23 present in the area indicated by the thick diagonal lines in FIG. 20 are an example of the non-detection area of the detector.

As illustrated in FIGS. 19 and 20, the area indicated by the thick diagonal lines in FIG. 20 is smaller than that indicated by the thick diagonal lines in FIG. 19. In other words, the sixth modification can increase the detection areas of the laser sensors 23 compared with the fifth modification.

### Seventh Modification

As described in the first embodiment, the control function 51 displays the display screen 421 for notifying the operator of the detection area DA on the display 42 (display panel 42a). In the present modification, the control function 51 displays a display screen for notifying the operator of a tube-placeable area on the display 42. The tube-placeable area is an example of the non-detection area.

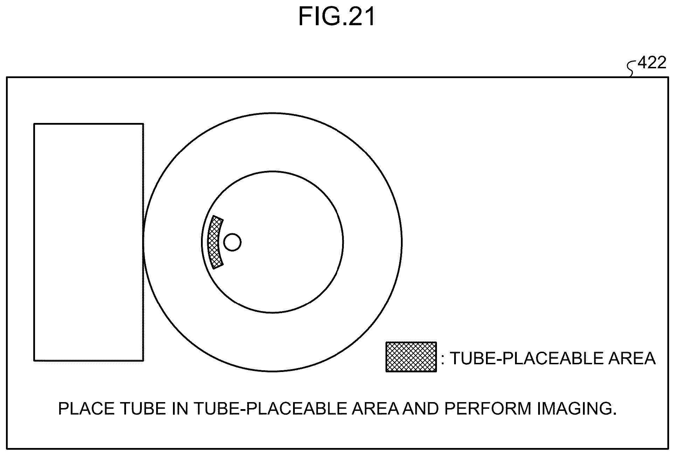

FIG. 21 is a view of an example of a display screen 422 for notifying the operator of the tube-placeable area according to a seventh modification. As illustrated in FIG. 21, the display screen 422 displays the area other than the detection area DA as the tube-placeable area. The display screen 422 also displays a message for urging the operator to place the tube in the tube-placeable area.

With this configuration, the operator can adjust the position of the tube so as to place the tube outside the detection area DA before starting the imaging of the subject P. By placing the tube outside the detection area DA, the X-ray CT apparatus 1 according to the present modification can prevent the operation of the scanner 20 from being stopped due to the tube erroneously determined to be the subject P.

### Eighth Modification

As described in the second embodiment, the detection function 56a determines whether the subject P or an object other than the subject P is detected depending on the number of laser sensors 23 that determine that the adjacent laser beam is blocked. In the present modification, the laser sensor 23 employs a cross-beam system, and it is determined whether the subject P or an object other than the subject P is detected according to the height of the object or the size of the area obstructed by the object.

The light emitters 24 according to the present modification emit laser beams such that the laser beams intersect between the light emitters 24 and the light receivers 26 in the vertical direction.

The detection function 56a according to the present modification identifies the height of the object that blocks the laser beams and the size of the area obstructed by the object according to how the laser beams are blocked (hereinafter referred to as a blocking pattern). The detection function 56a determines whether the subject P or an object other than the subject P is detected.

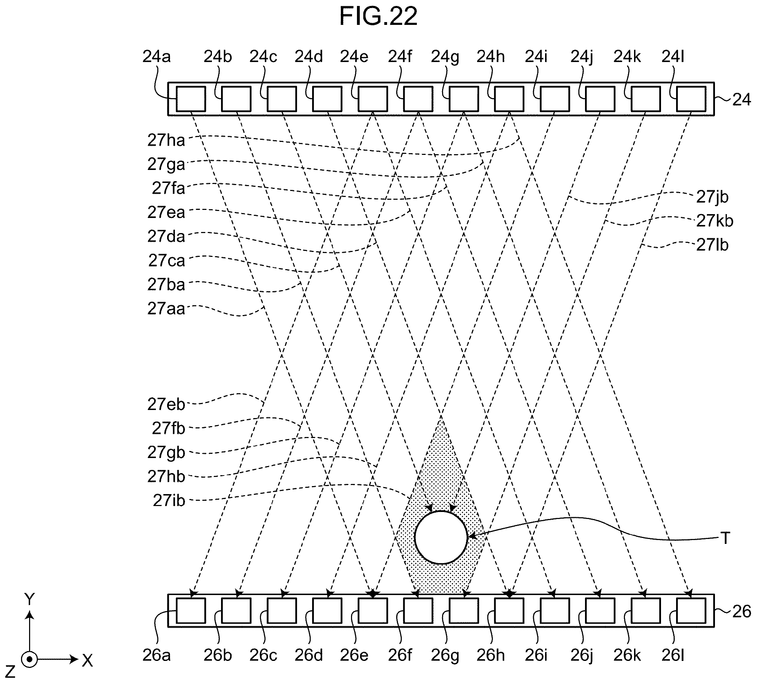

FIG. 22 is a diagram for explaining an example of the processing for determining whether the subject or an object other than the subject is detected according to an eighth modification. FIG. 22 is a developed view of the light emitters 24a to 24l and the light receivers 26a to 26l arranged in a circle similarly to FIGS. 14 and 15.

As illustrated in FIG. 22, the light emitters 24a to 24h emit laser beams 27aa to 27ha diagonally downward toward the light receivers 26e to 26l, respectively. Similarly, the light emitters 24e to 24l emit laser beams 26eb to 27lb diagonally downward toward the light receivers 26a to 26h, respectively.

It is assumed that the light emitters 24a to 24d emit laser beams diagonally downward toward the light receivers 26i to 26l, respectively, which is not illustrate to simplify the drawings. Similarly, it is assumed that the light emitters 24i to 24l emit laser beams diagonally downward toward the light receivers 26a to 26d, respectively.

With this configuration, the laser beams 27 emitted by the light emitters 24a to 24h toward the light receivers 26e to 26l intersect the laser beams 27 emitted by the light emitters 24e to 24l toward the light receivers 26a to 26h between the light emitters 24 and the light receivers 26 in the vertical direction.

By crossing the laser beams 27 emitted from the light emitters 24 in this manner, the detection function 56a can identify the position in the vertical direction of the object (height of the object) that blocks the laser beams 27 and the area obstructed by the object based on the combination of the laser beams 27 determined to be blocked.

In the example in FIG. 22, the tube T blocks the laser beam 27ca and the laser beam 27jb. In this example, it can be found out that the tube T is present at a low position near the light receivers 26 in the vertical direction. It can also be found out that the area indicated by the dot pattern is obstructed by the tube T.

Typically, tubes are placed at a position near the floor surface in the vertical direction. Therefore, the detection function 56a according to the present modification determines that an object other than the subject P is detected in the detection area DA when it identifies that the object is present at a position lower than a predetermined height based on the blocking pattern.

As the size of the object that blocks the laser beams increases, the size of the area obstructed by the object increases. Therefore, the detection function 56a according to the present modification determines that the subject P is detected in the detection area DA when it identifies that an area equal to or larger than a predetermined size is obstructed by the object based on the blocking pattern.

When the determination based on the height of the object is different from the determination based on the size of the obstructed area, the detection function 56a may determine in advance which one takes priority and determine whether the subject P or an object other than the subject P is detected in the detection area DA based on the priority.

The detection function 56a may determine that the subject P is detected in the detection area DA when it determines that the subject P is detected in the detection area DA in at least one of the determination by the height of the object and the determination by the size of the obstructed area. Alternatively, the detection function 56a may determine that the subject P is detected in the detection area DA only when it determines that the subject P is detected in the detection area DA in both the determination by the height of the object and the determination by the size of the obstructed area.

In the present modification, the operator can set the detection area DA based on the results of detection of an object other than the subject P by the detection function 56a. The following describes the workflow for setting the detection area DA according to the present modification.

For example, the operator determines the position of the tube during a main scan of the subject P before performing the main scan of the subject P. Subsequently, the operator actually places the tube according to the determination and causes the X-ray CT apparatus 1a to detect the tube by the laser sensors 23. The operator then determines (sets) the detection area DA by determining the laser sensor 23 to be disabled based on the results of detection of the tube.

In the present modification, it can be determined whether to continue or stop the operation of the scanner 20 based on the results of detection of the object including the subject P by the detection function 56a while the scanner 20 is operating (scanning the subject P).

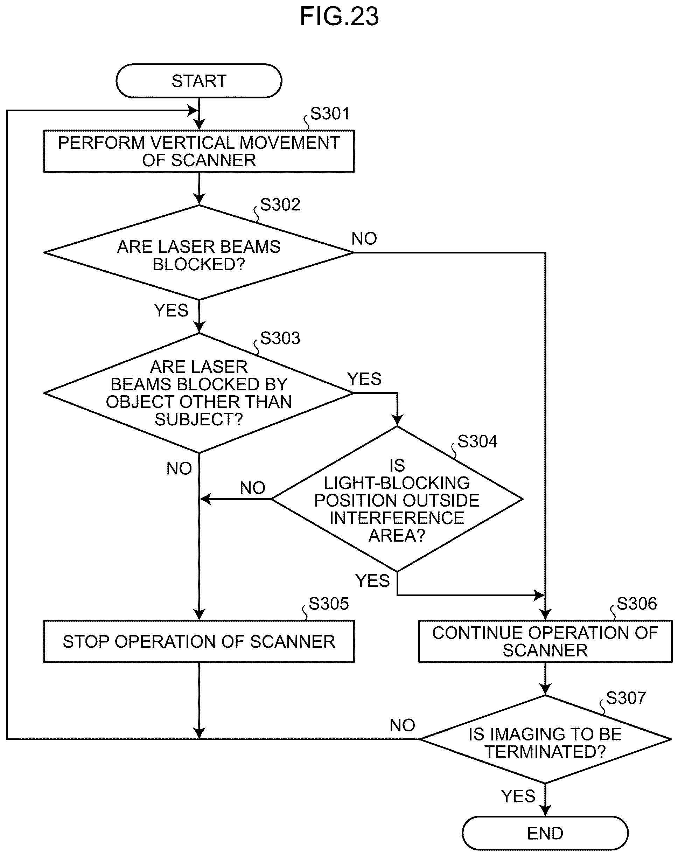

The following describes the processing for determining whether to continue or stop the operation of the scanner 20. FIG. 23 is a flowchart of an example of the processing performed by the X-ray CT apparatus 1a according to the eighth modification.

First, the control function 51 performs vertical movement of the scanner 20 (Step S301). For example, the control function 51 controls the scanner drive apparatus 22 to perform the vertical movement of the scanner 20. Subsequently, the detection function 56a determines whether the laser beams are blocked (Step S302) as in the processing at Step S205 in FIG. 16. If the laser beams are not blocked (No at Step S302), the process proceeds to Step S306, which will be described later.

By contrast, if the laser beams are blocked (Yes at Step S302), the detection function 56a determines whether the laser beams are blocked by an object other than the subject P (Step S303).

For example, if the detection function 56a identifies that an area smaller than the predetermined size is obstructed by an object based on the blocking pattern, it determines that the laser beams are blocked by an object other than the subject P. By contrast, if the detection function 56a identifies that an area equal to or larger than the predetermined size is obstructed by an object based on the blocking pattern, it determines that the laser beams are blocked by the subject P.

If it is determined that the laser beams are blocked by the subject P (No at Step S303), the process proceeds to Step S305, which will be described later. By contrast, if it is determined that the laser beams are blocked by an object other than the subject P (Yes at Step S303), the detection function 56a determines whether the light-blocking position is outside an interference area (Step S304).

For example, if the detection function 56a identifies that the object is present at a position lower than the predetermined height based on the blocking pattern, it determines that the light-blocking position is outside the interference area. By contrast, if the detection function 56a identifies that the object is present at a position higher than the predetermined height based on the blocking pattern, it determines that the light-blocking position is in the interference area.

If it is determined that the light-blocking position is outside the interference area (Yes at Step S304), the control function 51 continues the operation of the scanner 20 (Step S306).

Subsequently, the control function 51 determines whether an instruction to terminate the imaging is received from the operator (Step S307) as in the processing at Step S113 in FIG. 12B. If an instruction to terminate the imaging is not received (No at Step S307), the process returns to Step S301. By contrast, if an instruction to terminate the imaging is received (Yes at Step S307), the present processing is terminated.

If it is determined that the light-blocking position is in the interference area at Step S304 (No at Step S304), the control function 51 stops the operation of the scanner 20 (Step S305), and the process returns to Step S301. For example, the control function 51 controls the scanner drive apparatus 22 to stop the vertical movement of the scanner 20.

The above has described an example where the X-ray CT apparatus 1a determines whether to continue or stop the operation of the scanner 20 based on the results of detection by the detection function 56a. The X-ray CT apparatus 1a, however, may perform control to switch the enabled/disabled state of the laser sensors 23 based on the results of detection by the detection function 56a similarly to the second embodiment described above.

According to the present modification, it can be more accurately determined whether the subject P or an object other than the subject P is detected in the detection area DA.

At least one of the embodiments described above can reduce the possibility of erroneously determining an object that may be present between the scanner and the floor surface as the subject and causing the apparatus to stop during imaging.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the inventions as defined by the appended claims. The accompanying claims are intended to cover such forms or modifications as would fall within the scope of the inventions.

### Notes

The following collectively describes various aspects of the present disclosure as notes.

### Note 1.

An X-ray CT apparatus comprising:
a scanner unit having an imaging system;
a support unit that supports the scanner unit and configured to move the scanner unit in a vertical direction; and
processing circuitry configured to:
   perform control to:
      define at least part of an area between an outer periphery of an opening into which a subject is inserted and the subject as a detection area and detect that an object is present in the detection area; and
      define the detection area included in an area in which movement of the subject is restricted as a non-detection area; and
   stop an operation of the scanner unit when it is detected that the object is present in the detection area excluding the non-detection area while the scanner unit is operating.

### Note 2.

The processing circuitry may perform control to define an area corresponding to an installation position of a subject support member for supporting the subject as the non-detection area in standing position imaging or sitting position imaging of the subject.

### Note 3.

The X-ray CT apparatus may further include a plurality of optical sensors provided to the scanner unit and configured to detect the object. The processing circuitry may perform control to detect that the object is present in the detection area based on a result of detection by each of the optical sensors and disable at least one optical sensor present in the non-detection area according to the installation position of the subject support member for supporting the subject out of the optical sensors.

### Note 4.

The processing circuitry may perform control to define an area including the optical sensor closest to the installation position of the subject support member alone out of the optical sensors as the non-detection area.

### Note 5.

The processing circuit may perform control to define the area behind the installation position of the subject support member out of the area including the optical sensors as the non-detection area.

### Note 6.

The optical sensors may be divided into at least two group areas according to positions at which the optical sensors are provided. The processing circuitry may perform control to define the group area closest to the installation position of the subject support member out of the area including the optical sensors as the non-detection area. Note 7.

The processing circuitry may display at least one of the detection area and the non-detection area on a display medium.

### Note 8.

The display medium may be a display unit of a display apparatus provided to the support unit, and the processing circuitry may display at least one of the detection area and the non-detection area on the display unit.

### Note 9.

The display medium may be at least one of an exterior of the scanner unit and a floor surface of an examination room in which the X-ray CT apparatus is installed. The processing circuitry may display at least one of the detection area and the non-detection area on the exterior or the floor surface using at least one of a plurality of light-emitting diodes (LEDs) provided to the exterior, a projector configured to project an image onto the exterior, and a display apparatus embedded in the floor surface.

### Note 10.

The processing circuitry may define an area opposite to a direction in which the subject is capable of moving during imaging as the non-detection area.

### Note 11.

An X-ray CT apparatus comprising:
a scanner unit having an imaging system;
a support unit that supports the scanner unit and configured to move the scanner unit in a vertical direction; and
processing circuitry configured to:
   define at least part of an area between an outer periphery of an opening into which a subject is inserted and the subject as a detection area and detect that the subject is present in the detection area or that an object other than the subject is present in the detection area; and
   stop an operation of the scanner unit when it is detected that the subject is present in the detection area while the scanner unit is operating, wherein
the processing circuitry does not perform control to stop the operation of the scanner unit when it is detected that the object other than the subject is present in the detection area.

### Note 12.

The X-ray CT apparatus may further include a plurality of optical sensors configured to detect the object, and the optical sensors may each comprise a light emitter configured to emit a laser beam toward a light receiver and the light receiver configured to receive the emitted laser beam. The processing circuitry may determine that at least one of the subject and the object other than the subject is detected in the detection area when at least one of the light receivers fails to receive the laser beam and identify whether the subject or the object other than the subject is detected in the detection area based on the number of light receivers that determine that the laser beam adjacent to the light receivers fails to be received.

### Note 13.

The light emitters of the optical sensors may each emit a plurality of the laser beams such that the laser beams intersect between each of the light emitters and each of the light receivers in the vertical direction. The processing circuitry may identify whether the subject or the object other than the subject is detected in the detection area based on a combination of the light receivers that fail to receive the laser beams.

### Note 14.

The processing circuitry may detect the object other than the subject based on the combination of the light receivers that fail to receive the laser beams in a non-detection area other than the detection area, and present an area around the scanner unit to an operator of the X-ray CT apparatus by dividing the area into "an area serving as the detection area and in which the object other than the subject is detected", "an area serving as the detection area and in which the object other than the subject is not detected", "an area serving as the non-detection area and in which the object other than the subject is detected", and "an area serving as the non-detection area and in which the object other than the subject is not detected".

### Note 15.

A control method by an X-ray CT apparatus comprising a scanner unit having an imaging system, and a support unit that supports the scanner unit and configured to move the scanner unit in a vertical direction, the control method comprising:
performing control to:
   defining at least part of an area between an outer periphery of an opening into which a subject is inserted and the subject as a detection area and detecting an object present in the detection area; and
   defining an area in which movement of the subject is restricted as a non-detection area; and
stopping an operation of the scanner unit when it is detected that the object is present in the detection area excluding the non-detection area while the scanner unit is operating.

### Note 16.

A non-transitory computer readable medium comprising instructions that cause a computer of an X-ray CT apparatus, the X-ray CT apparatus comprising a scanner unit having an imaging system, and a support unit that supports the scanner unit and configured to move the scanner unit in a vertical direction, to execute:
performing control to:
   defining at least part of an area between an outer periphery of an opening into which a subject is inserted and the subject as a detection area and detecting an object present in the detection area; and
   defining an area in which movement of the subject is restricted as a non-detection area; and
stopping an operation of the scanner unit when it is detected that the object is present in the detection area excluding the non-detection area while the scanner unit is operating.

## Claims

1. An X-ray CT apparatus (1) comprising:
a scanner unit (20) having an imaging system;
a support unit (103) that supports the scanner unit and configured to move the scanner unit in a vertical direction;
a detection unit (56) configured to define at least part of an area between an outer periphery of an opening into which a subject is inserted and the subject as a detection area and detect that an object is present in the detection area;
a detection control unit (55) configured to perform control to define the detection area included in an area in which movement of the subject is restricted as a non-detection area; and
an operation control unit (51) configured to stop an operation of the scanner unit when it is detected that the object is present in the detection area excluding the non-detection area while the scanner unit is operating.

2. The X-ray CT apparatus (1) according to claim 1, wherein
the detection control unit (55) performs control to define an area corresponding to an installation position of a subject support member (70) for supporting the subject as the non-detection area in standing position imaging or sitting position imaging of the subject.

3. The X-ray CT apparatus (1) according to claim 2, further comprising:
a plurality of optical sensors (23) provided to the scanner unit (20) and configured to detect the object, wherein
the detection unit (56)
detects that the object is present in the detection area based on a result of detection by each of the optical sensors (23), and
performs control to disable at least one optical sensor present in the non-detection area according to the installation position of the subject support member (70) for supporting the subject out of the optical sensors.

4. The X-ray CT apparatus (1) according to claim 3, wherein
the detection control unit (55) performs control to define an area including the optical sensor closest to the installation position of the subject support member (70) alone out of the optical sensors (23) as the non-detection area.

5. The X-ray CT apparatus (1) according to claim 3, wherein
the detection control unit (55) performs control to define the area behind the installation position of the subject support member (70) out of the area including the optical sensors (23) as the non-detection area.

6. The X-ray CT apparatus (1) according to claim 3, wherein
the optical sensors (23) are divided into at least two group areas according to positions at which the optical sensors are provided, and
the detection control unit (55) performs control to define the group area closest to the installation position of the subject support member (70) out of the area including the optical sensors as the non-detection area.

7. The X-ray CT apparatus (1) according to claim 1, further comprising:
a display control unit (51) configured to display at least one of the detection area and the non-detection area on a display medium.

8. The X-ray CT apparatus (1) according to claim 7, wherein
the display medium is a display unit (42) of a display apparatus provided to the support unit, and
the display control unit (51) displays at least one of the detection area and the non-detection area on the display unit (42).

9. The X-ray CT apparatus (1) according to claim 7, wherein
the display medium is at least one of an exterior of the scanner unit (20) and a floor surface of an examination room in which the X-ray CT apparatus is installed, and
the display control unit (51) displays at least one of the detection area and the non-detection area on the exterior or the floor surface using at least one of a plurality of light-emitting diodes (LEDs) provided to the exterior, a projector configured to project an image onto the exterior, and a display apparatus embedded in the floor surface.

10. The X-ray CT apparatus (1) according to claim 1, wherein
the detection control unit (51) defines an area opposite to a direction in which the subject is capable of moving during imaging as the non-detection area.

11. An X-ray CT apparatus (1) comprising:
a scanner unit (20) having an imaging system;
a support unit (103) that supports the scanner unit and configured to move the scanner unit in a vertical direction; and
a detection unit (56) configured to define at least part of an area between an outer periphery of an opening into which a subject is inserted and the subject as a detection area and detect that the subject is present in the detection area or that an object other than the subject is present in the detection area; and
an operation control unit (51) configured to stop an operation of the scanner unit when it is detected that the subject is present in the detection area while the scanner unit is operating, wherein
the operation detection control unit (51) does not perform control to stop the operation of the scanner unit (20) when it is detected that the object other than the subject is present in the detection area.

12. The X-ray CT apparatus (1) according to claim 11, further comprising:
a plurality of optical sensors (23) configured to detect the object, wherein
the optical sensors each comprise
a light emitter (24) configured to emit a laser beam toward a light receiver (26), and
the light receiver configured to receive the emitted laser beam,
the detection unit (56) determines that at least one of the subject and the object other than the subject is detected in the detection area when at least one of the light receivers (26) fails to receive the laser beam, and
the detection unit (56) identifies whether the subject or the object other than the subject is detected in the detection area based on the number of light receivers (26) that determine that the laser beam adjacent to the light receivers fails to be received.

13. The X-ray CT apparatus (1) according to claim 12, wherein
the light emitters (24) of the optical sensors each emit a plurality of the laser beams such that the laser beams intersect between each of the light emitters and each of the light receivers (26) in the vertical direction, and
the detection unit (56) identifies whether the subject or the object other than the subject is detected in the detection area based on a combination of the light receivers that fail to receive the laser beams.

14. The X-ray CT apparatus (1) according to claim 13, wherein
the detection unit (56) detects the object other than the subject based on the combination of the light receivers (26) that fail to receive the laser beams in a non-detection area other than the detection area, the apparatus further comprising:
a presentation unit (51) configured to present an area around the scanner unit to an operator of the X-ray CT apparatus by dividing the area into "an area serving as the detection area and in which the object other than the subject is detected", "an area serving as the detection area and in which the object other than the subject is not detected", "an area serving as the non-detection area and in which the object other than the subject is detected", and "an area serving as the non-detection area and in which the object other than the subject is not detected".

15. A control method by an X-ray CT apparatus (1) comprising a scanner unit (20) having an imaging system, and a support unit (103) that supports the scanner unit (20) and configured to move the scanner unit in a vertical direction, the control method comprising:
defining at least part of an area between an outer periphery of an opening into which a subject is inserted and the subject as a detection area and detecting an object present in the detection area; and
performing control to define an area in which movement of the subject is restricted as a non-detection area; and
stopping an operation of the scanner unit when it is detected that the object is present in the detection area excluding the non-detection area while the scanner unit is operating.
